# EUROPEAN PATENT APPLICATION

(11) **EP 4 775 568 A1**
(43) Date of publication of application: **15.07.2026**
(21) Application number: 24862962.8
(22) Date of filing: 05.06.2024
(51) Int. Cl.: C07D 231/56, A61K 31/416, A61K 31/4439, A61K 31/437, A61P 17/06, C07D 401/04, C07D 409/04, C07D 405/04, C07D 403/04, C07D 471/04

(54) **NOVEL COMPOUND AND USE THEREOF FOR TREATING PSORIASIS**

(30) Priority: 05.09.2023 KR 20230117763
(71) Applicant: Gwangju Institute of Science and Technology, Gwangju 61005 (KR)
(72) Inventor: KIM, Yong Chul, Gwangju 61005 (KR); KO, Bong Ki, Gwangju 61005 (KR)
(74) Representative: dompatent
(86) International application number: PCT/KR2024/007686
(87) International publication number: WO 2025/053379

(57) **Abstract**

The present invention relates to a novel compound and the use thereof for the treatment of psoriasis. The novel compound of the present invention exhibits inhibitory activity against chemokine-like receptor 1 (CMKLR1), thereby exhibiting excellent preventive and therapeutic effects on psoriasis.

## Description

### [Technical Field]

The present invention relates to a novel compound and the use thereof for the treatment of psoriasis.

### [Background Art]

Psoriasis is one of the chronic immune diseases characterized by symptoms including skin erythema and scaling. Various factors, including genetics, the environment, and irritation, have been implicated as causes. While not life-threatening, it may relapse at any time and may lead to secondary diseases such as arthritis, metabolic syndrome, and depression, and is therefore regarded as a serious skin disease. Current treatment options for symptom relief include topical treatments such as steroid ointments and immunomodulators, as well as oral treatments such as retinoids and methotrexate. However, these treatments are not perfect and only provide symptom relief. Therefore, the development of a safe psoriasis treatment with novel targets is urgently needed.

### [Summary of Invention]

### [Problems to be Solved by Invention]

An object of the present invention is to provide a novel compound having excellent inhibitory activity against Chemokine-like receptor 1 (CMKLR1).

Another object of the present invention is to provide a pharmaceutical composition exhibiting excellent effects in preventing or treating psoriasis by inhibiting CMKLR1.

### [Means for Solving Problems]

1. A compound represented by Formula 1 below or a pharmaceutically acceptable salt thereof:

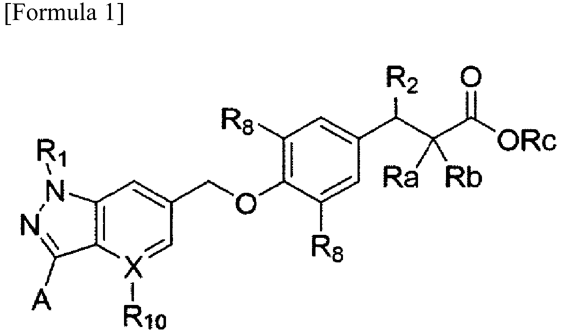

(in Formula 1, A is a substituted or unsubstituted 5- to 10-membered aryl or heteroaryl; R₁ is C₁ to C₄ alkyl, C₃ to C₆ cycloalkyl, acetyl, benzyl, or methyl substituted with C₃ to C₆ cycloalkyl; R₂ is C₁ to C₄ alkyl or C₃ to C₆ cycloalkyl; R₈ is hydrogen or halogen; Rₐ and R_{b} are each independently hydrogen or methyl; R_{c} is hydrogen or C₁ to C₄ alkyl; X is carbon or nitrogen; and R₁₀ is absent, hydrogen, halogen, or methyl unsubstituted or substituted with halogen).
2. The compound or a pharmaceutically acceptable salt thereof according to above 1, wherein A is substituted or unsubstituted phenyl, pyridine, benzo[b]thiophene, or 1H-tetrazole.
3. The compound or a pharmaceutically acceptable salt thereof according to above 1, wherein the substituent of A is C₁ to C₃ alkyl, C₁ to C₅ alkoxy, -OH, -CF₃, -CN, -CH₂OH, -NH₂, -CH=NOH, halogen, phenyl, -NO₂, -OCH₂CH=CHC(=O)OH, -OCH₂CH=CH₂, - C(=O)OH, -NHC(=O)R₉, or dioxane, and R₉ is C₂ to C₄ alkyl or alkenyl.
4. The compound or a pharmaceutically acceptable salt thereof according to above 1, wherein A is substituted or unsubstituted phenyl, and the substituent is selected from the group consisting of C₁ to C₃ alkyl, C₁ to C₅ alkoxy, -OH, -CF₃, -CN, -CH₂OH, -NH₂, - CH=NOH, halogen, phenyl, -NO₂, -OCH₂CH=CHC(=O)OH, -OCH₂CH=CH₂, -C(=O)OH, -NHC(=O)R₉ (wherein R₉ is C2 to C4 alkyl or alkenyl), or dioxane.
5. The compound or a pharmaceutically acceptable salt thereof according to above 1, wherein the compound represented by Formula 1 is any one selected from the group consisting of the following compounds:

| Compoun d | Structure | Compoun d | Structure |
|---|---|---|---|
| 1 | 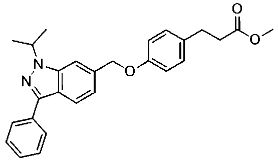 | 2 | 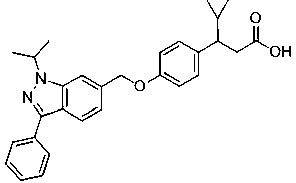 |
| 3 | 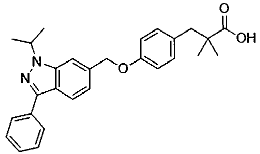 | 4 | 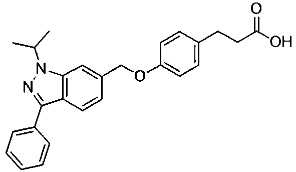 |
| 5 | 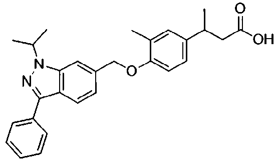 | 6 | 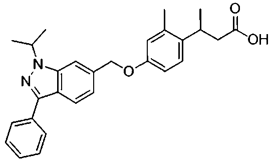 |
| 7 | 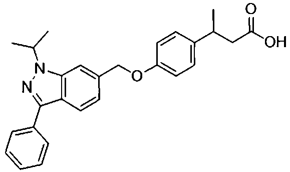 | 8 | 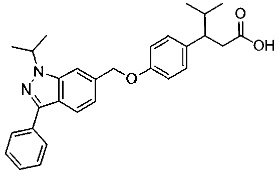 |
| 9 | 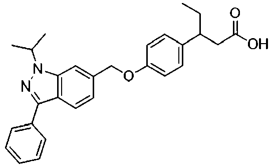 | 10 | 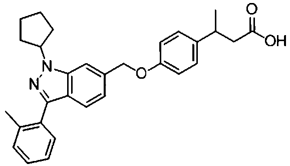 |

| Compoun d | Structure | Compoun d | Structure |
|---|---|---|---|
| 11 | 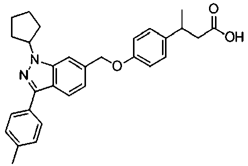 | 12 | 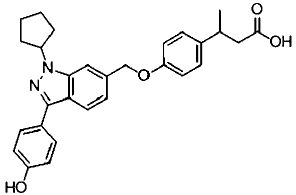 |
| 13 | 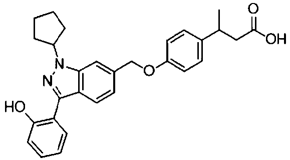 | 14 | 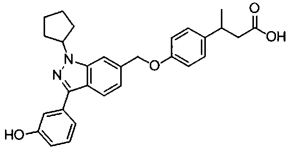 |
| 15 | 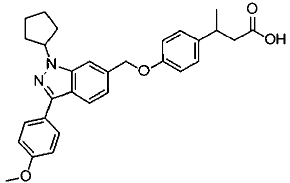 | 16 | 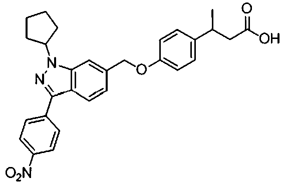 |
| 17 | 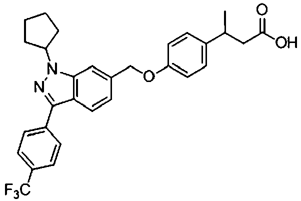 | 18 | 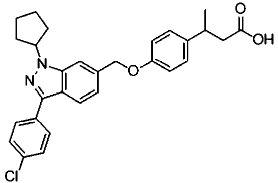 |
| 19 | 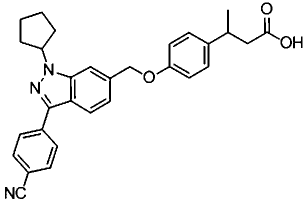 | 20 | 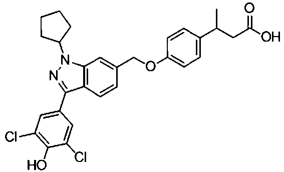 |

| Compoun d | Structure | Compoun d | Structure |
|---|---|---|---|
| 21 | 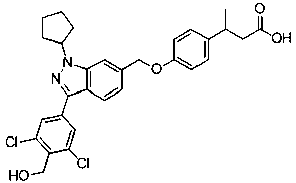 | 22 | 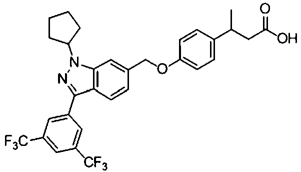 |
| 23 | 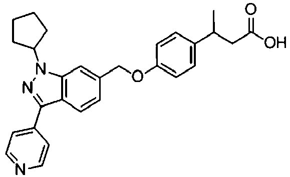 | 24 | 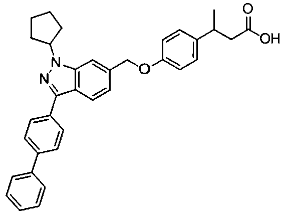 |
| 25 | 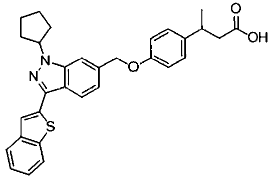 | 26 | 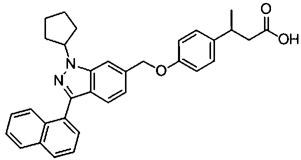 |
| 27 | 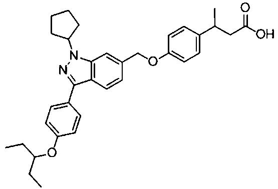 | 28 | 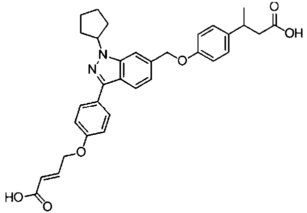 |
| 29 | 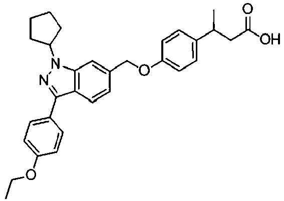 | 30 | 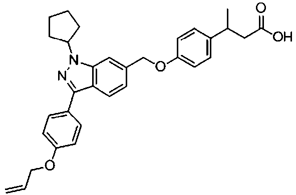 |

| Compoun d | Structure | Compoun d | Structure |
|---|---|---|---|
| 31 | 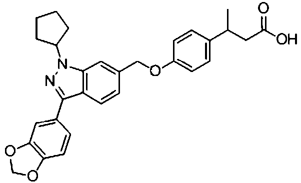 | 32 | 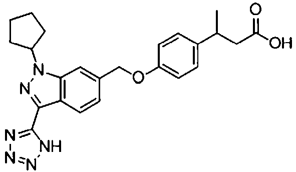 |
| 33 | 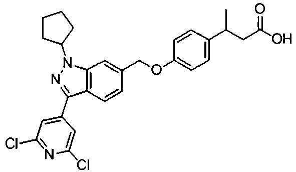 | 34 | 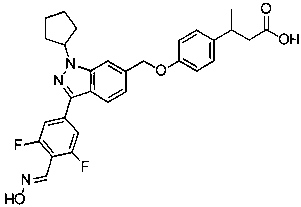 |
| 35 | 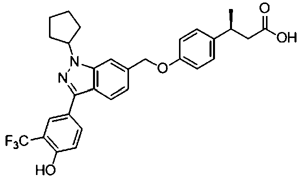 | 36 | 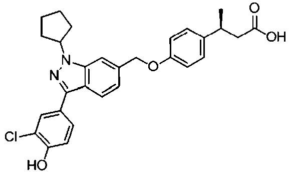 |
| 37 | 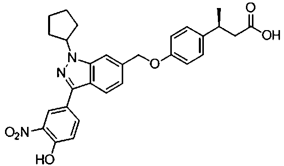 | 38 | 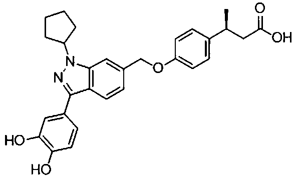 |
| 39 | 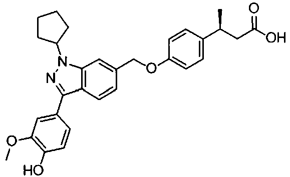 | 40 | 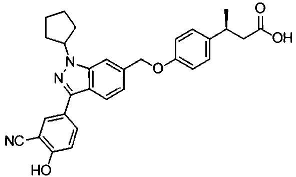 |

| Compoun d | Structure | Compoun d | Structure |
|---|---|---|---|
| 41 | 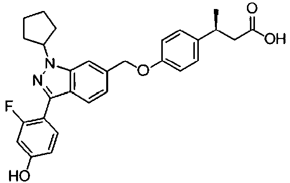 | 42 | 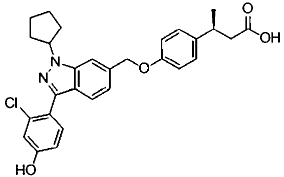 |
| 43 | 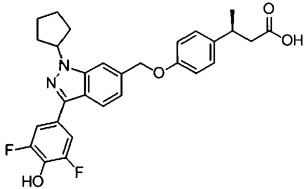 | 44 | 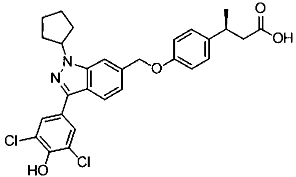 |
| 45 | 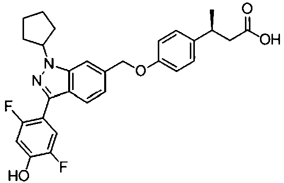 | 46 | 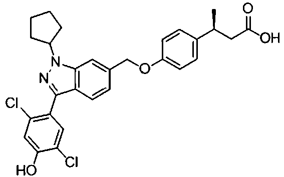 |
| 47 | 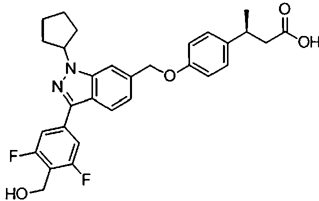 | 48 | 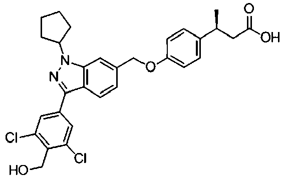 |
| 49 | 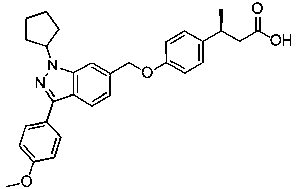 | 50 | 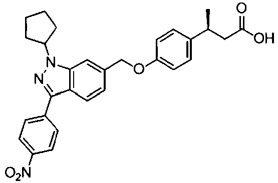 |

| Compoun d | Structure | Compoun d | Structure |
|---|---|---|---|
| 51 | 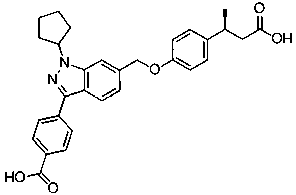 | 52 | 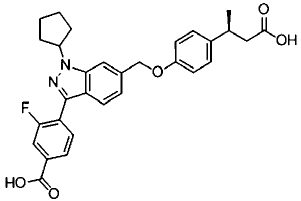 |
| 53 | 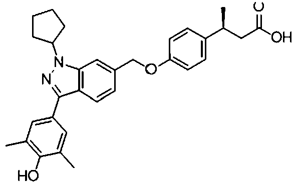 | 54 | 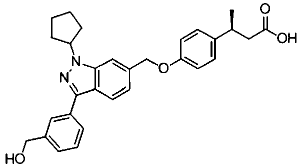 |
| 55 | 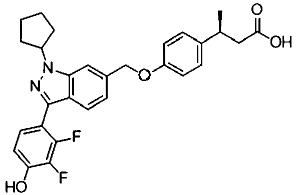 | 56 | 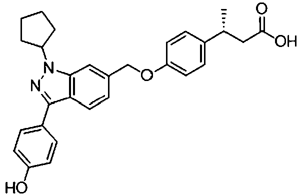 |
| 57 | 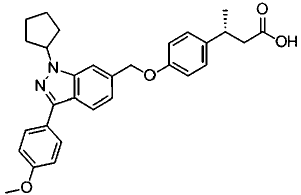 | 58 | 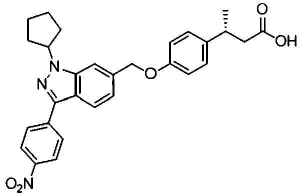 |
| 59 | 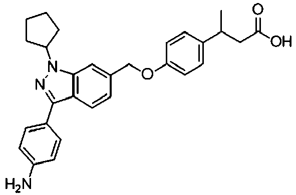 | 60 | 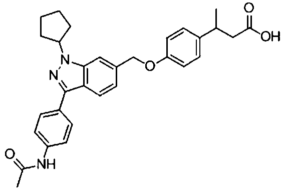 |

| Compoun d | Structure | Compoun d | Structure |
|---|---|---|---|
| 61 | 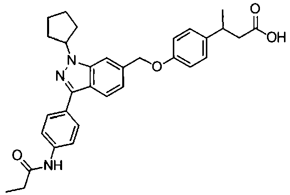 | 62 | 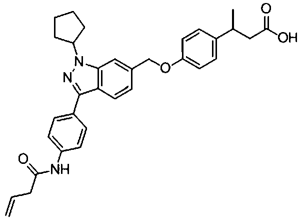 |
| 63 | 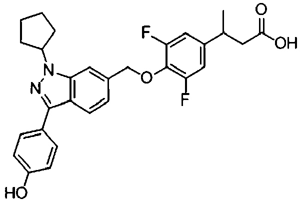 | 64 | 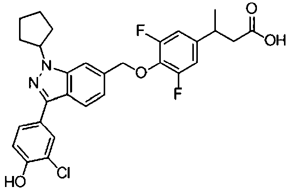 |
| 65 | 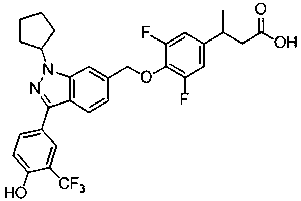 | 66 | 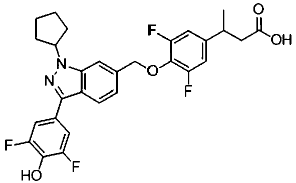 |
| 67 | 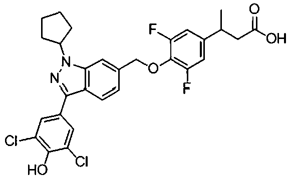 | 68 | 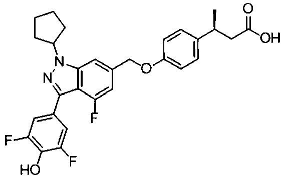 |
| 69 | 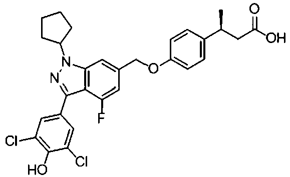 | 70 | 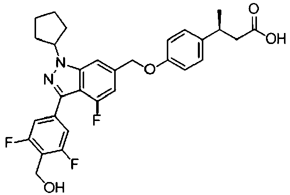 |

| Compoun d | Structure | Compoun d | Structure |
|---|---|---|---|
| 71 | 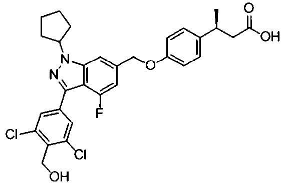 | 72 | 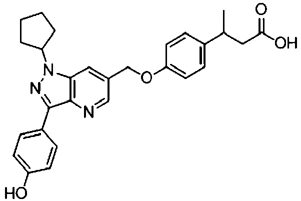 |
| 73 | 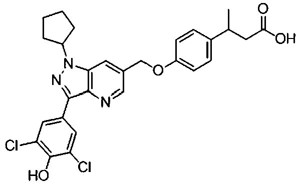 | 74 | 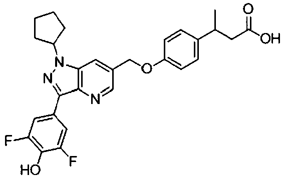 |
| 75 | 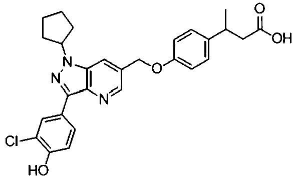 | 76 | 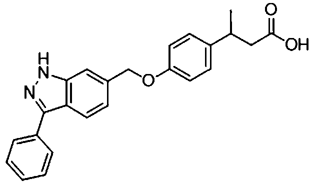 |
| 77 | 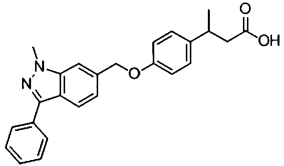 | 78 | 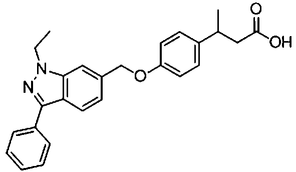 |
| 79 | 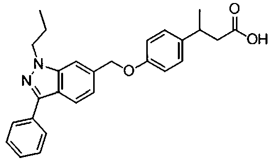 | 80 | 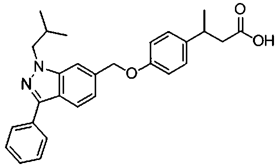 |

| Compoun d | Structure | Compoun d | Structure |
|---|---|---|---|
| 81 | 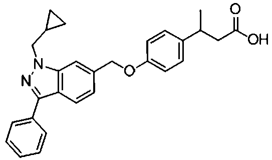 | 82 | 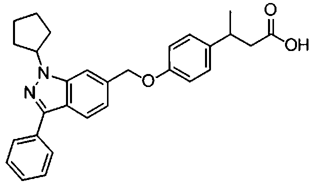 |
| 83 | 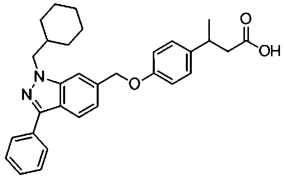 | 84 | 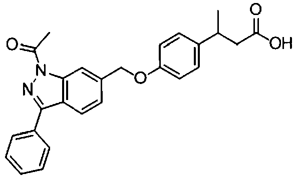 |
| 85 | 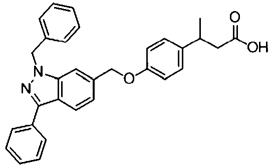 | 86 | 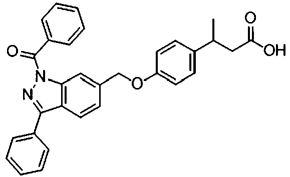 |

6. A pharmaceutical composition for preventing or treating psoriasis, including the compound of any one of the above 1 to 5 or a pharmaceutically acceptable salt thereof.

### [Advantageous effects]

The compound of the present invention exhibits excellent CMKLR1 inhibitory activity.

The pharmaceutical composition of the present invention exhibits excellent effects in the prevention or treatment of psoriasis.

### [Brief Description of Drawings]

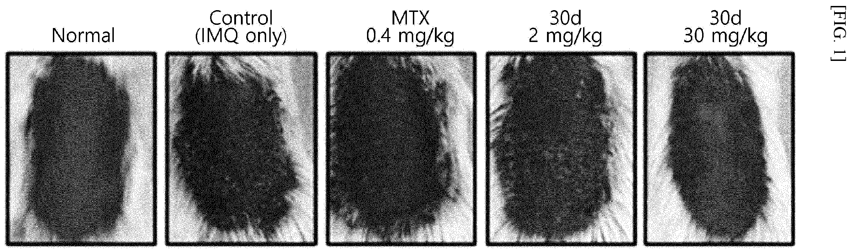

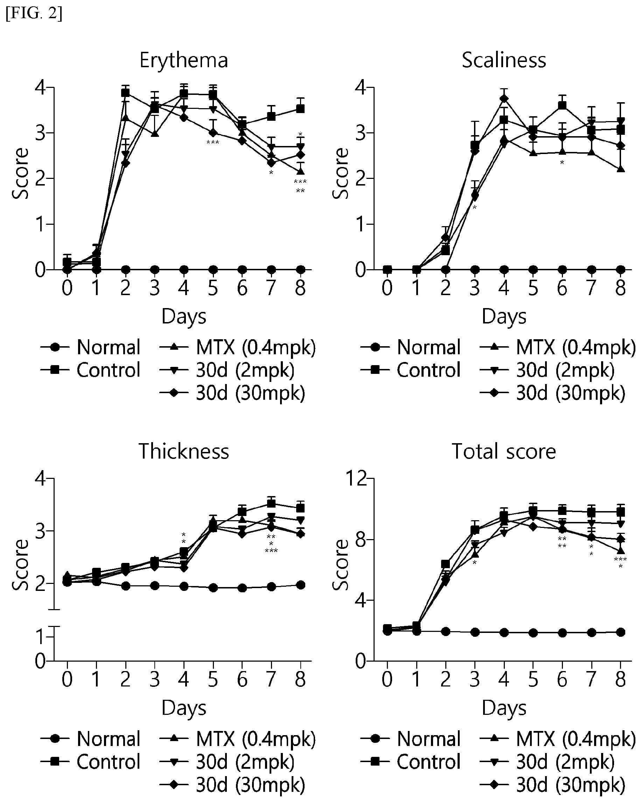

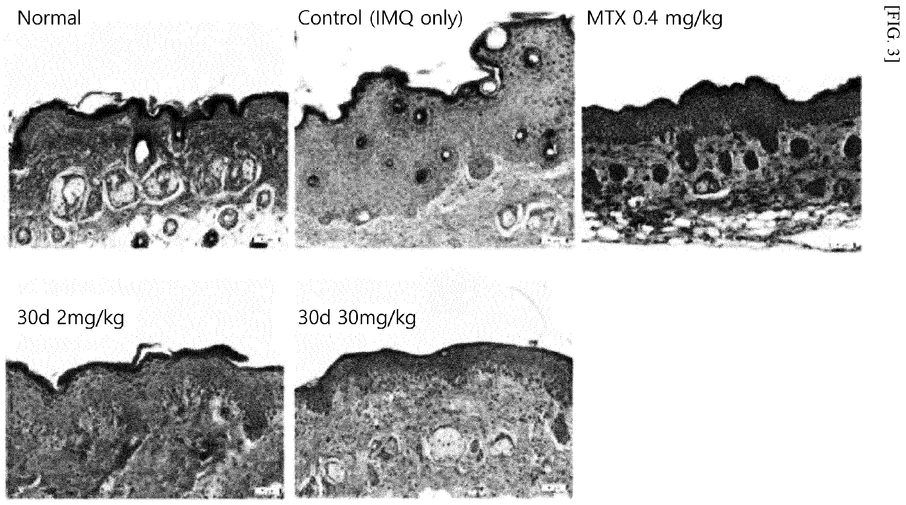

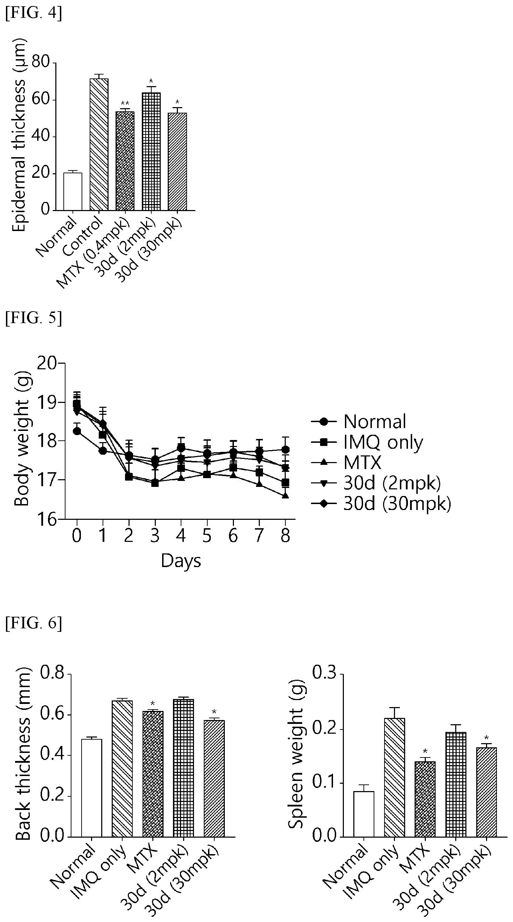

FIGS. 1 to 6 illustrate the results of administering compound 30d (= Compound 71), corresponding to Formula 1 of the present invention, to a mouse psoriasis model induced with imiquimod.
FIG. 1 is a series of photos showing backs of each mouse on day 7 of the experiment.
FIG. 2 is a series of graphs showing changes in PASI scores during the experiment.
FIG. 3 is a series of images showing the results of H&E staining of dorsal skin tissue of mice.
FIG. 4 is a graph showing the epidermal thickness of each experimental group.
FIG. 5 is a graph showing changes in body weight of each experimental group during the experimental period.
FIG. 6 is a series of graphs showing dorsal skin thickness and spleen weight of each experimental group.

### [Mode for Carrying out Invention]

The present invention relates to a novel compound having excellent inhibitory activity against Chemokine-like receptor 1 (CMKLR1) and the use thereof for the prevention or treatment of psoriasis.

The present invention provides a compound represented by Formula 1 below or a pharmaceutically acceptable salt thereof:

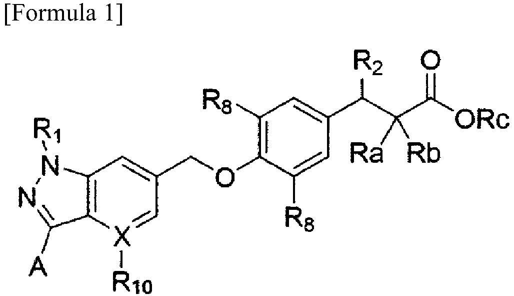

In Formula 1, A is a substituted or unsubstituted 5- to 10-membered aryl or heteroaryl;
R₁ is C₁ to C₄ alkyl, C₃ to C₆ cycloalkyl, acetyl, benzyl, or methyl substituted with C₃ to C₆ cycloalkyl;
R₂ is C₁ to C₄ alkyl or C₃ to C₆ cycloalkyl;
R₈ is hydrogen or halogen;
Rₐ and R_{b} are each independently hydrogen or methyl;
R_{c} is hydrogen or C₁ to C₄ alkyl;
X is carbon or nitrogen; and R₁₀ is absent, hydrogen, halogen, or methyl unsubstituted or substituted with halogen.

As used herein, the term "alkyl" refers to a linear or branched saturated hydrocarbon group containing the indicated number of carbon atoms, such as methyl, ethyl, n-propyl, or isopropyl.

As used herein, the term "alkenyl" refers to a linear or branched unsaturated hydrocarbon group containing the indicated number of carbon atoms and at least one carbon-carbon double bond, such as - CH₂CH=CH₂ or -CH₂CH₂CH=CH₂.

As used herein, the term "cycloalkyl" refers to a monocyclic saturated hydrocarbon ring containing the indicated number of ring atoms, and C₃ to C₅ cycloalkyl may include, for example, cyclopropyl, cyclobutyl, and cyclopentyl.

As used herein, the term "alkoxy" refers to -O-(alkyl), where alkyl is as defined above. Examples of alkoxy include -OCH₃, -OCH₂CH₃, -O(CH₂)₂CH₃, -O(CH₂)₃CH₃, - O(CH₂)₄CH₃, -O(CH₂)₅CH₃ and the like.

As used herein, the term "halogen" refers to fluorine (F), chlorine (Cl), bromine (Br), or iodine (I).

As used herein, the term "acyl" refers to a substituted carbonyl group "-C(=O)-R_{c}."

As used herein, the term "aryl" refers to an aromatic hydrocarbon ring, which may be monocyclic or polycyclic. For example, a 5- to 10-membered aryl may include phenyl, naphthyl, or biphenyl.

As used herein, the term "heteroaryl" refers to an aromatic ring containing one or more heteroatoms selected from N, O, and S as ring-forming atoms, and may be monocyclic or polycyclic. For example, a 5- to 10-membered heteroaryl may include pyridine, benzo[b]thiophene, 1H-tetrazole and the like.

In Formula 1, A may be a substituted or unsubstituted 5- to 10-membered aryl or heteroaryl, preferably a substituted or unsubstituted phenyl, pyridine, benzo[b]thiophene, or 1H-tetrazole, and more preferably a substituted or unsubstituted phenyl. The 5- to 10-membered aryl or heteroaryl may be substituted with 1 to 5 substituents. The substituent may be C₁ to C₃ alkyl, C₁ to C₅ alkoxy, -OH, -CF₃, -CN, -CH₂OH, -NH₂, -CH=NOH, halogen, phenyl, -NO₂, -OCH₂CH=CHC(=O)OH, -OCH₂CH=CH₂, -C(=O)OH or - NHC(=O)R₉, or two substituents may be combined to form a 1,3-dioxane.

R₁ may be C₁ to C₄ alkyl, C₃ to C₆ cycloalkyl, acetyl, benzyl, or methyl substituted with a C₃ to C₆ cycloalkyl, preferably isopropyl or cyclopentyl.

R₂ may be C₁ to C₄ alkyl or C₃ to C₆ cycloalkyl, preferably methyl, and more preferably methyl bonded such that the chiral center of the carbon to which R₂ is attached is in the R configuration. For example, R₂ may be methyl bonded as in compounds 68 to 70 prepared in Preparative Example 7.

R₈ may be hydrogen or halogen, preferably hydrogen or fluorine.

R₉ may be C₂ to C₄ alkyl or alkenyl.

Rₐ and R_{b} may each independently be hydrogen or methyl.

R_{c} may be hydrogen or C₁ to C₄ alkyl.

Preferably, the compound represented by Formula 1 may be a compound represented by Formula 2 below.

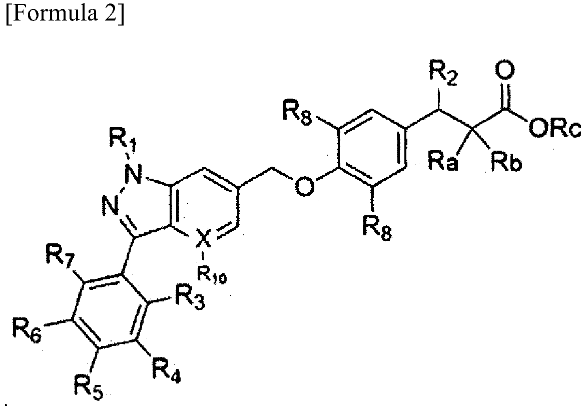

In Formula 2, R₁, R₂, R₈, Rₐ, R_{b}, R_{c}, and X are the same as defined in Formula 1 above.

R₃ to R₇ may each independently be hydrogen, C₁ to C₃ alkyl, C₁ to C₅ alkoxy,-OH, -CF₃, -CN, -CH₂OH, -NH₂, -CH=NOH, halogen, phenyl, -NO₂, - OCH₂CH=CHC(=O)OH, -OCH₂CH=CH₂, -C(=O)OH, or -NHC(=O)R₉. R₉ is the same as in Formula 1.

R₆ and R₇ together may form a 1,3-dioxane.

When X is carbon, R₁₀ may be hydrogen, halogen (F, Cl, Br, or I), or methyl substituted with halogen (CF₃, CCl₃, CBR₃, or CI₃).

When X is nitrogen, R₁₀ is absent.

The compound represented by Formula 1 may be any one selected from the group consisting of compounds 1 to 86 below.

| Compoun d | Structure | Compoun d | Structure |
|---|---|---|---|
| 1 | 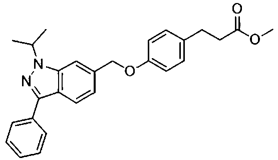 | 2 | 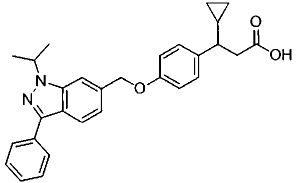 |
| 3 | 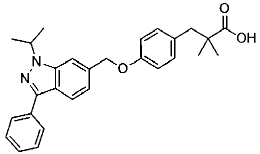 | 4 | 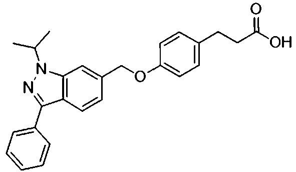 |
| 5 | 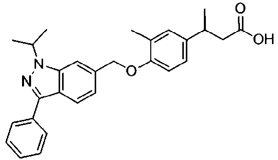 | 6 | 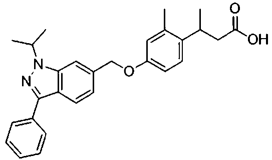 |
| 7 | 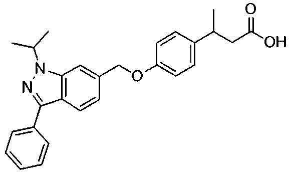 | 8 | 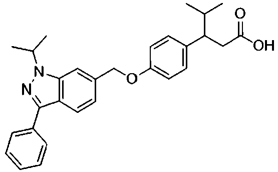 |
| 9 | 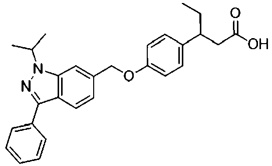 | 10 | 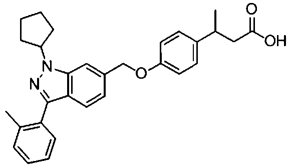 |

| Compoun d | Structure | Compoun d | Structure |
|---|---|---|---|
| 11 | 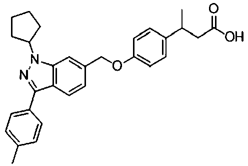 | 12 | 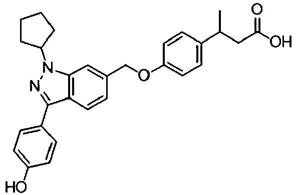 |
| 13 | 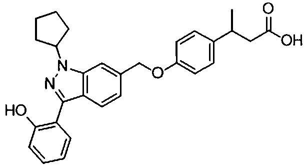 | 14 | 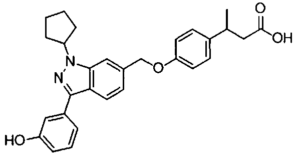 |
| 15 | 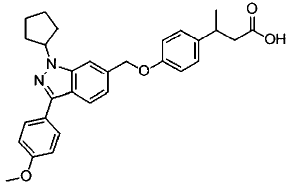 | 16 | 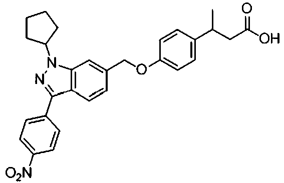 |
| 17 | 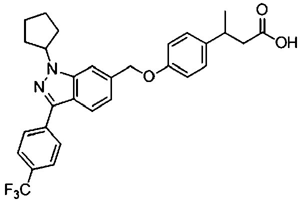 | 18 | 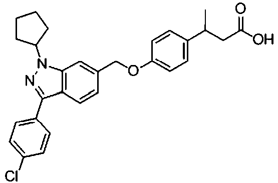 |
| 19 | 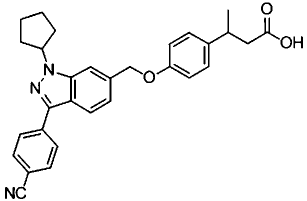 | 20 | 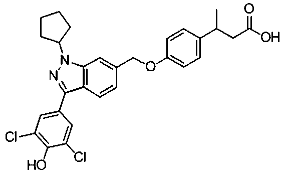 |

| Compoun d | Structure | Compoun d | Structure |
|---|---|---|---|
| 21 | 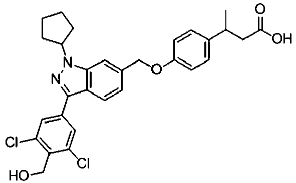 | 22 | 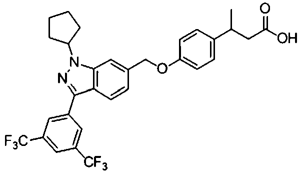 |
| 23 | 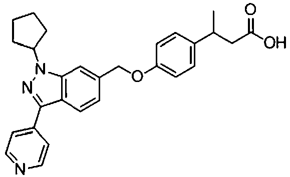 | 24 | 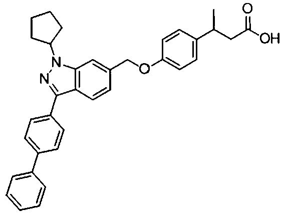 |
| 25 | 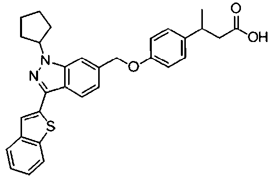 | 26 | 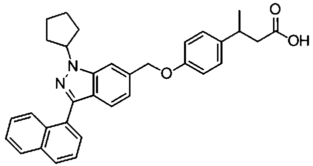 |
| 27 | 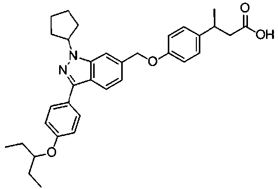 | 28 | 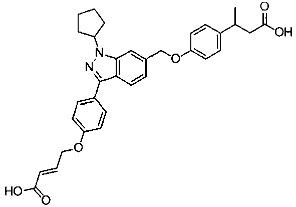 |
| 29 | 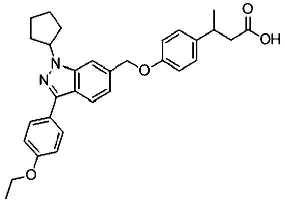 | 30 | 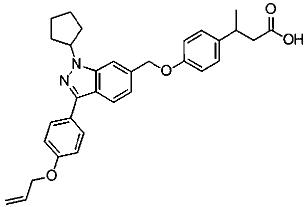 |

| Compoun d | Structure | Compoun d | Structure |
|---|---|---|---|
| 31 | 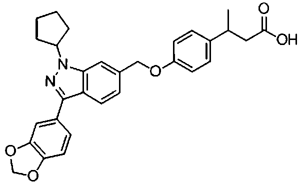 | 32 | 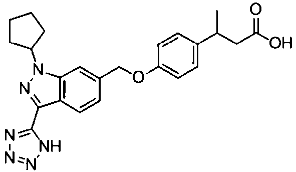 |
| 33 | 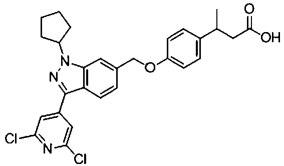 | 34 | 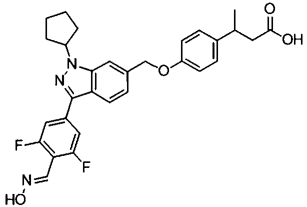 |
| 35 | 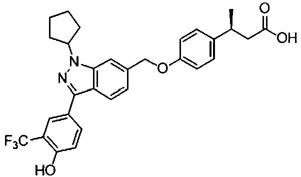 | 36 | 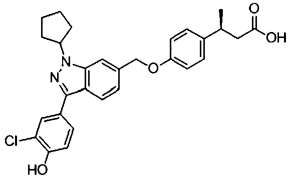 |
| 37 | 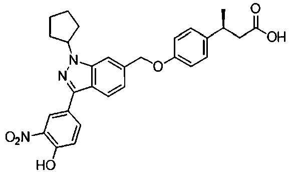 | 38 | 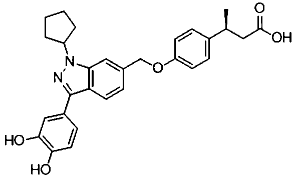 |
| 39 | 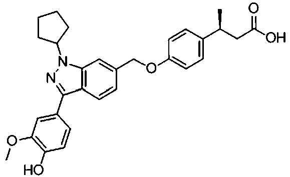 | 40 | 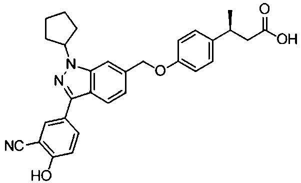 |

| Compoun d | Structure | Compoun d | Structure |
|---|---|---|---|
| 41 | 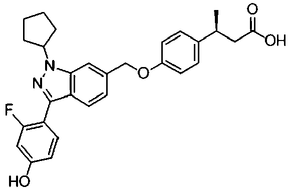 | 42 | 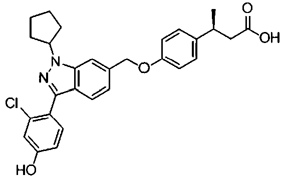 |
| 43 | 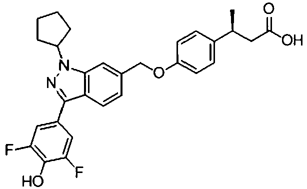 | 44 | 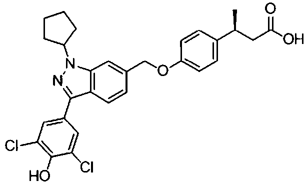 |
| 45 | 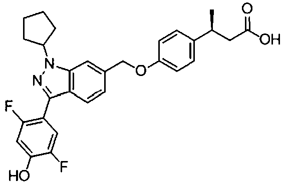 | 46 | 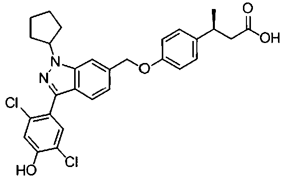 |
| 47 | 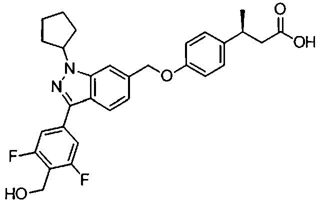 | 48 | 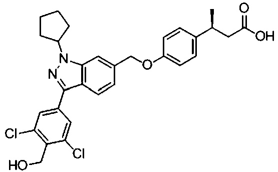 |
| 49 | 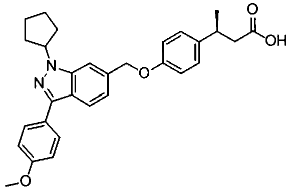 | 50 | 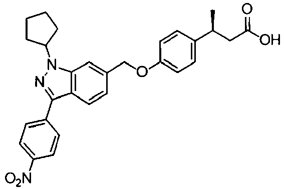 |

| Compoun d | Structure | Compoun d | Structure |
|---|---|---|---|
| 51 | 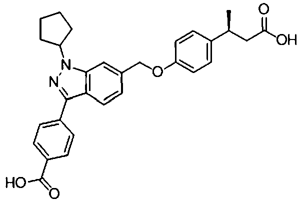 | 52 | 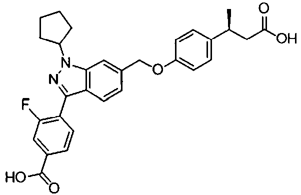 |
| 53 | 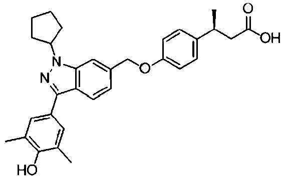 | 54 | 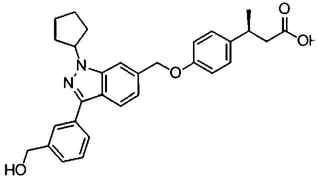 |
| 55 | 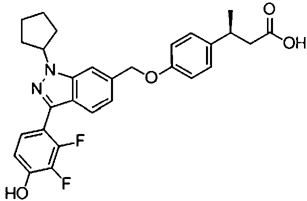 | 56 | 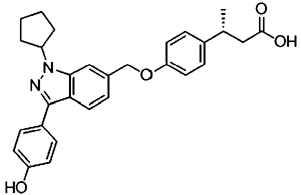 |
| 57 | 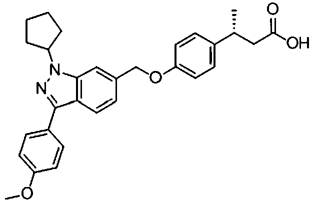 | 58 | 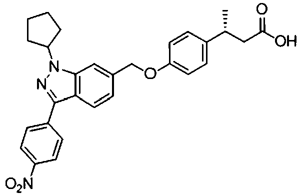 |
| 59 | 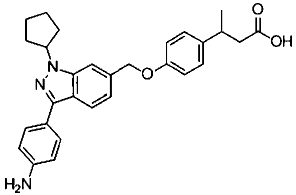 | 60 | 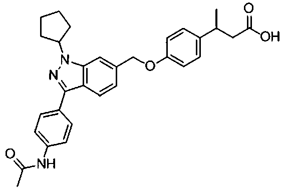 |

| Compoun d | Structure | Compoun d | Structure |
|---|---|---|---|
| 61 | 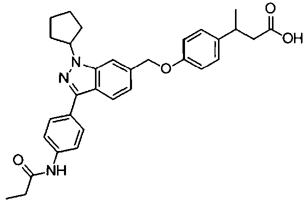 | 62 | 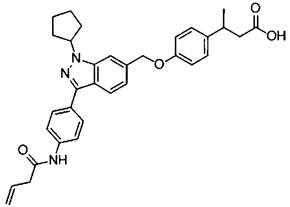 |
| 63 | 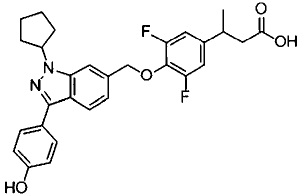 | 64 | 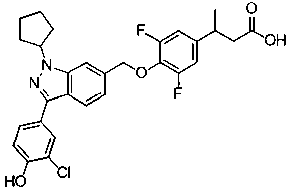 |
| 65 | 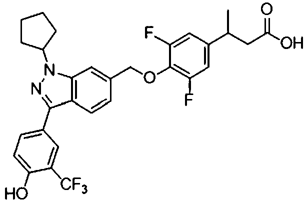 | 66 | 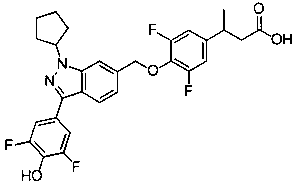 |
| 67 | 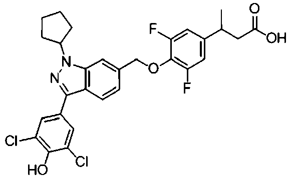 | 68 | 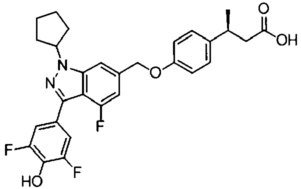 |
| 69 | 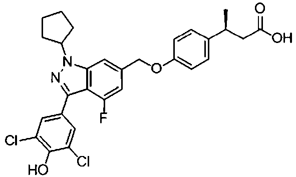 | 70 | 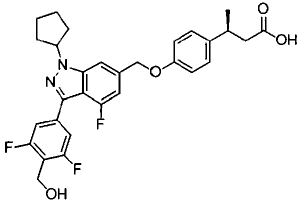 |

| Compoun d | Structure | Compoun d | Structure |
|---|---|---|---|
| 71 | 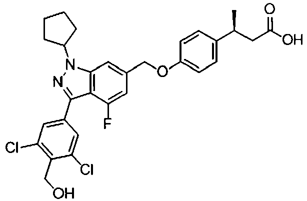 | 72 | 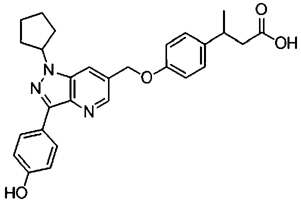 |
| 73 | 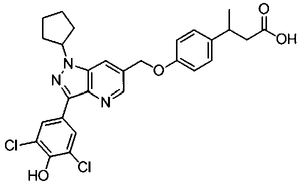 | 74 | 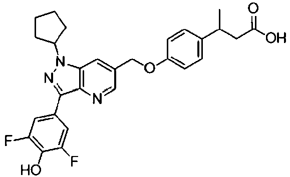 |
| 75 | 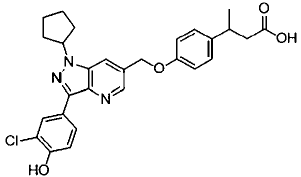 | 76 | 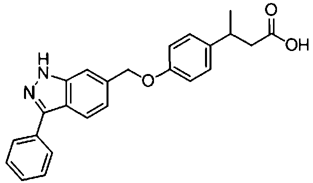 |
| 77 | 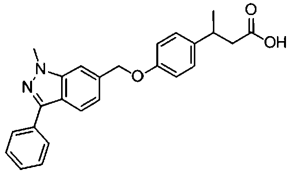 | 78 | 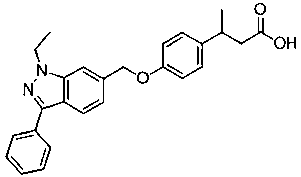 |
| 79 | 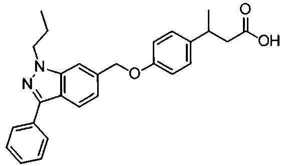 | 80 | 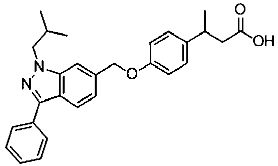 |

| Compoun d | Structure | Compoun d | Structure |
|---|---|---|---|
| 81 | 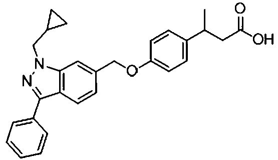 | 82 | 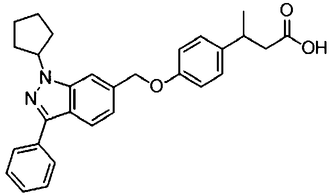 |
| 83 | 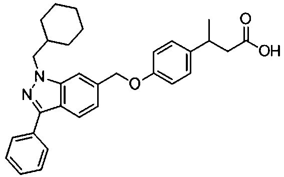 | 84 | 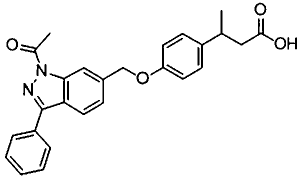 |
| 85 | 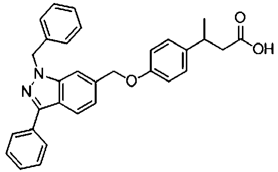 | 86 | 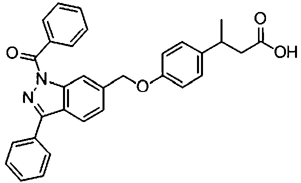 |

The compound represented by Formula 1 has excellent Chemokine-like receptor 1 (CMKLR1) inhibition activity.

The compound represented by Formula 1 inhibits Chemokine-like receptor 1 (CMKLR1) and may be used for the prevention or treatment of psoriasis.

The present invention provides a pharmaceutical composition for preventing or treating psoriasis, including the compound represented by Formula 1 described above or a pharmaceutically acceptable salt thereof.

The term "psoriasis" as used herein refers to an inflammatory disease that occurs in the skin or joints due to an immune system disorder, causing problems such as disfigurement, scaling, erythematous plaques, and pain. Psoriasis may include any one or more diseases selected from psoriatic arthritis, guttate psoriasis, pustular psoriasis, erythrodermic psoriasis, scalp psoriasis, nail psoriasis, and enthesitis.

Pharmaceutically acceptable salts may be, for example, acid addition salts, base addition salts or metal salts.

The acid addition salts may be formed from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and dicarboxylates, phenyl-substituted alkanoates, hydroxyalkanoates, alkane dioates, aromatic acids, and aliphatic and aromatic sulfonic acids. These pharmaceutically non-toxic salts may include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propylate, oxalate, malonate, succinate, suberate, sebacate, fumarate, maleate, but-2-yne-1,4-dioate, hexane-1,6-dioate, benzoate, chlorobenzoate, methylbenzoate, dinitrobenzoate, hydroxybenzoate, methoxybenzoate, phthalate, terephthalate, benzene sulfonate, toluene sulfonate, chlorobenzene sulfonate, xylene sulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, or mandelate. For example, the acid addition salt of the compound represented by Formula 1 may be obtained by dissolving the compound in an excess amount of aqueous acid solution and precipitating the salt using a hydrated organic solvent such as methanol, ethanol, acetone, or acetonitrile.

The metal salt may be a sodium, potassium, or calcium salt. The metal salt may be prepared using a base; for example, alkali metal or alkaline earth metal salts may be obtained by dissolving the compound in an excess amount of an alkali metal hydroxide or alkaline earth metal hydroxide solution, filtering the undissolved compound salt, and evaporating and/or drying the filtrate.

The pharmaceutical composition of the present invention may be in the form of a capsule, tablet, granule, injection, ointment, powder or beverage.

The pharmaceutical composition of the present invention may be formulated and used in oral dosage forms such as powders, granules, capsules, tablets, and aqueous suspensions, as well as external preparations, suppositories and injections.

The pharmaceutical composition of the present invention may contain an active ingredient alone, or may further include one or more pharmaceutically acceptable carriers, excipients, or diluents.

The pharmaceutical composition of the present invention may include a pharmaceutically acceptable carrier. The pharmaceutically acceptable carrier may include binders, lubricants, disintegrants, excipients, solubilizers, dispersants, stabilizers, suspending agents, coloring agents, and flavoring agents for oral administration. In additon, for injections, buffering agents, preservatives, soothing agents, solubilizing agents, isotonic agents, and stabilizers may be mixed and used. Further, for topical administration, bases, excipients, lubricants, and preservatives may be used.

The formulation of the pharmaceutical composition of the present invention may be prepared in various ways by mixing the composition with a pharmaceutically acceptable carrier. For example, when administered orally, it may be prepared in the forms of tablets, troches, capsules, elixirs, suspensions, syrups, or wafers. Further, in the case of an injection, it may be prepared in a unit-dose ampoule or a multi-dose form. In addition, the formulation of the pharmaceutical composition of the present invention may be prepared as a solution, suspension, tablet, capsule, sustained-release formulation, or the like.

The carriers, excipients, and diluents for formulation may include, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, fillers, anti-caking agents, lubricants, wetting agents, flavoring agents, emulsifiers, or preservatives.

The administration route of the pharmaceutical composition of the present invention may include, for example, oral, intravenous, intramuscular, intra-arterial, intramedullary, intrathecal, intracardiac, transdermal, subcutaneous, intraperitoneal, intranasal, intestinal, topical, sublingual, or rectal, but is not limited thereto.

The pharmaceutical composition of the present invention may be administered orally or parenterally, and when administered parenterally, topical administration or injection routes such as intraperitoneal, rectal, subcutaneous, intravenous, intramuscular, or intrathoracic injection may be selected.

The dosage of the pharmaceutical composition of the present invention may vary depending on the condition and weight of the patient, the degree of the disease, the form of the drug, and the route and duration of administration, but may be appropriately selected by those skilled in the art.

For example, the pharmaceutical composition of the present invention may be administered at 0.0001 to 1000 mg/kg or 0.001 to 500 mg/kg per day. Further, the pharmaceutical composition of the present invention may be administered once a day, or may be administered in divided doses.

Hereinafter, the present invention will be described in more detail through examples.

### Preparative Examples

### Preparative Example 1. Synthesis of Compounds 1 to 9

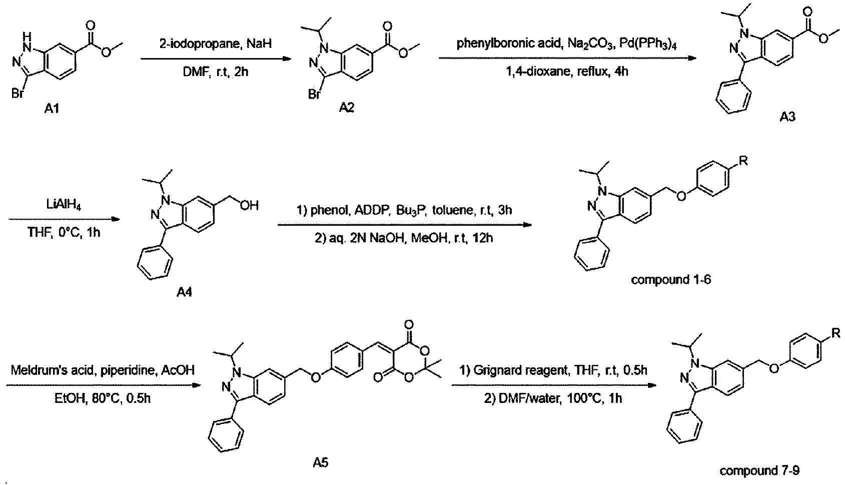

### (1) Synthesis of methyl 3-bromo-1-isopropyl-1H-indazole-6-carboxylate (A2)

Methyl 3-bromo-1H-indazole-6-carboxylate (A1) (20 mg, 0.078 mmol) was dissolved in DMF (0.5 mL), and NaH (60% in mineral oil, 0.156 mmol) was added. 2-iodopropane (11 µL, 0.118 mmol) was then added to the reaction mixture, and the mixture was stirred at room temperature for 2 h. The reaction mixture was poured into water and extracted with ethyl acetate and saturated aqueous NH₄Cl solution. The combined extracts were dried over Na₂SO₄, filtered, and concentrated under reduced pressure to yield a crude product. The crude product was purified by silica gel column chromatography (hexane:ethyl acetate = 25:1) to obtain Compound A2 as a white solid (8 mg, 35% yield).

¹H NMR (CDCl₃, 400 MHz): δ 8.21 (s, 1H), 7.85 (dd, *J* = 1.22, 8.55 Hz, 1H), 7.65 (d, *J* = 8.55 Hz, 1H), 4.91 (td, *J=* 6.71, 13.43 Hz, 1H), 3.99 (s, 3H), 1.62 (d, *J* = 6.71 Hz, 6H). MS (ESI): [M + H]⁺ = 298.82

### (2) Synthesis of methyl 1-isopropyl-3-phenyl-1H-indazole-6-carboxylate (A3)

A2 (18 mg, 0.061 mmol), phenylboronic acid (8.2 mg, 0.066 mmol), Pd(PPh₃)₄ (7 mg, 0.0061 mmol), and Na₂CO₃ (19 mg, 0.183 mmol) were dissolved in 1,4-dioxane (2 mL) and water (1 mL), and the mixture was stirred under reflux for 4 h. The reaction mixture was evaporated under reduced pressure. The residue was dissolved in water and extracted with ethyl acetate. The combined extracts were dried over Na₂SO₄, filtered, and concentrated under reduced pressure to yield a crude product. The crude product was purified by silica gel column chromatography (hexane:ethyl acetate = 35:1) to obtain Compound A3 as a white solid (15 mg, 87% yield).

¹H NMR (CDCl₃, 400 MHz): δ 8.23 - 8.27 (m, 1H), 8.05 (dd, *J* = 0.76, 8.70 Hz, 1H), 7.95 - 8.01 (m, 2H), 7.86 (dd, *J =* 1.22, 8.55 Hz, 1H), 7.49 - 7.55 (m, 2H), 7.38 - 7.44 (m, 1H), 4.93 - 5.05 (m, 1H), 4.00 (s, 3H), 1.68 (d, *J=* 6.71 Hz, 6H). MS (ESI): [M + H]⁺ =295.11

### (3) Synthesis of 1-isopropyl-3-phenyl-1H-indazol-6-yl)methanol (A4)

A3 (15 mg, 0.051 mmol) was dissolved in THF (1 mL) and cooled to 0°C. Next, 1.0 M lithium aluminum hydride (102 µL, 0.102 mmol) dissolved in THF was added dropwise, and the mixture was stirred at 0°C for 1 h. The reaction mixture was poured into saturated aqueous NH₄Cl solution and extracted with ethyl acetate. The combined extracts were dried over Na₂SO₄, filtered, and concentrated under reduced pressure to yield a crude product. The crude product was purified by silica gel column chromatography (CHCl₃:MeOH = 50:1) to obtain Compound A4 as a white solid (12 mg, 88% yield).

¹H NMR (CDCl₃, 400 MHz): δ 7.94 - 8.02 (m, 3H), 7.47 - 7.55 (m, 3H), 7.35 - 7.43 (m, 1H), 7.18 (dd, *J =* 1.22, 8.24 Hz, 1H), 4.89 - 4.95 (m, 1H), 4.88 (s, 2H), 1.66 (d, *J= 6.71* Hz, 6H)). MS (ESI): [M + H]⁺ = 267.14

### (4) Synthesis of methyl 3-(4-((1-isopropyl-3-phenyl-1H-indazol-6-yl)methoxy)phenyl)propanoate (Compound 1)

A3 (0.038 mmol) and methyl 3-(4-hydroxyphenyl)propanoate (0.056 mmol) were dissolved in toluene. Then, 1,1'-(azodicarbonyl)dipiperidine (0.076 mmol) and tributylphosphine (0.076 mmol) were added to the reaction mixture, and the mixture was stirred at room temperature for 3 h. The reaction mixture was filtered and washed with hexane. The filtrate was concentrated under reduced pressure to yield a crude product. The crude product was purified by silica gel column chromatography (hexane:ethyl acetate = 50:1) to obtain Compound 1 as a white solid.

¹H NMR (CDCl₃, 400 MHz): δ 8.02 (dd, *J=* 0.76, 8.39 Hz, 1H), 7.96 - 8.00 (m, 2H), 7.57 (s, 1H), 7.47 - 7.54 (m, 2H), 7.39 (tt, *J =* 1.26, 7.44 Hz, 1H), 7.23 - 7.27 (m, 1H), 7.11 - 7.15 (m, 2H), 6.95 - 6.98 (m, 2H), 5.22 (s, 2H), 4.91 (quin, *J =* 6.64 Hz, 1H), 2.51 - 2.61 (m, 2H), 1.59 - 1.69 (m, 8H), 0.92 - 0.97 (m, 3H). MS (ESI): [M + H]⁺ = 385.4

### (5) Synthesis of 3-cyclopropyl-3-(4-((1-isopropyl-3-phenyl-1H-indazol-6-yl) methoxy) phenyl) propanoic acid (Compound 2)

A3 (20 mg, 0.075 mmol) and methyl 3-cyclopropyl-3-(4-hydroxyphenyl) propanoate (26 mg, 0.112 mmol) were dissolved in toluene (1 mL). Next, 1,1'-(azodicarbonyl) dipiperidine (38 mg, 0.150 mmol) and tributylphosphine (37 µL, 0.150 mmol) were added to the reaction mixture, and the mixture was stirred at room temperature for 3 h. The reaction mixture was then concentrated under reduced pressure, and the residue was dissolved in MeOH (4 mL). Then, 2 N aqueous NaOH solution (1 mL) was added dropwise, and the mixture was stirred at room temperature for 12 h. The reaction mixture was then neutralized to pH 7 with 1 N aqueous NaOH solution and evaporated under reduced pressure. The residue was purified by silica gel column chromatography (CHC13:MeOH) to obtain Compound 2 as a white solid (8 mg, 24% yield).

¹H NMR (CDCl₃, 400 MHz): δ 8.02 (d, *J* = 8.24 Hz, 1H), 7.98 (dd, *J* = 1.37, 8.39 Hz, 2H), 7.57 (s, 1H), 7.47 - 7.53 (m, 2H), 7.36 - 7.41 (m, 1H), 7.23 - 7.27 (m, 2H), 6.85 - 6.96 (m, 3H), 5.22 (s, 2H), 4.84 - 4.98 (m, 1H), 2.80 (dd, *J =* 5.04, 7.48 Hz, 2H), 2.32 - 2.43 (m, 1H), 1.65 (d, *J =* 6.71 Hz, 6H), 0.95 - 1.09 (m, 1H), 0.55 - 0.63 (m, 1H), 0.38 - 0.46 (m, 1H), 0.26 - 0.33 (m, 1H), 0.11 - 0.22 (m, 1H). MS (ESI): [M + H]⁺ = 455.11

### (6) Synthesis of 3-(4-((1-isopropyl-3-phenyl-1H-indazol-6-yl)methoxy)phenyl)-2,2-dimethylpropanoic acid (Compound 3)

Compound 3 as a white solid (21% yield) was synthesized using A3 and methyl 3-(4-hydroxyphenyl)-2,2-dimethylpropanoate in the same manner as described in (5) above.

¹H NMR (CDCl₃, 400 MHz): δ 8.00 (dd, *J* = 0.61, 8.24 Hz, 1H), 7.95 - 7.98 (m, 2H), 7.54 (s, 1H), 7.47 - 7.52 (m, 2H), 7.35 - 7.42 (m, 1H), 7.23 (dd, *J =* 1.37, 8.39 Hz, 1H), 7.09 - 7.14 (m, 2H), 6.93 - 6.97 (m, 2H), 5.20 (s, 2H), 4.88 (quin, *J* = 6.71 Hz, 1H), 2.85 (s, 2H), 1.64 (d, *J=* 6.40 Hz, 6H), 1.21 (s, 6H). MS (ESI): [M + H]⁺ = 443.09

### (7) Synthesis of 3-(4-((1-isopropyl-3-phenyl-1H-indazol-6-yl)methoxy)phenyl)propanoic acid (Compound 4)

Compound 4 as a white solid (18% yield) was synthesized using A3 and methyl 3-(4-hydroxyphenyl)propanoate in the same manner as described in (5) above.

¹H NMR (CDCl₃, 400 MHz): δ 7.99 - 8.03 (m, 1H), 7.95 - 7.99 (m, 2H), 7.55 (s, 1H), 7.47 - 7.54 (m, 2H), 7.35 - 7.42 (m, 1H), 7.24 (dd, *J =* 1.22, 8.24 Hz, 1H), 7.14 - 7.19 (m, 2H), 6.95 - 7.00 (m, 2H), 5.21 (s, 2H), 4.85 - 4.93 (m, 1H), 2.91 - 2.96 (m, 2H), 2.64 - 2.69 (m, 2H), 1.65 (d, *J=* 6.41 Hz, 6H). MS (ESI): [M + H]⁺ = 414.98

### (8) Synthesis of 3-(4-((1-isopropyl-3-phenyl-1H-indazol-6-yl)methoxy)-3-methylphenyl)butanoic acid (Compound 5)

Compound 5 as a white solid (45% yield) was synthesized using A3 and methyl 3-(4-hydroxy-3-methylphenyl)butanoate in the same manner as described in (5) above.

### (9) Synthesis of 3-(4-((1-isopropyl-3-phenyl-1H-indazol-6-yl)methoxy)-2-methylphenyl)butanoic acid (Compound 6)

Compound 6 as a white solid (22% yield) was synthesized using A3 and methyl 3-(4-hydroxy-2-methylphenyl)butanoate in the same manner as described in (5) above.

### (10) Synthesis of 4-((1-isopropyl-3-phenyl-1H-indazol-6-yl)methoxy)benzaldehyde (A5 precursor)

Compound A5 precursor as a white solid (82% yield) was synthesized using A3 and 4-hydroxybenzaldehyde in the same manner as described in (4) above.

¹H NMR (CDCl₃, 400 MHz): δ 8.02 (d, *J =* 8.24 Hz, 1H), 7.98 (dd, *J=* 1.37, 8.39 Hz, 2H), 7.57 (s, 1H), 7.47 - 7.53 (m, 2H), 7.36 - 7.41 (m, 1H), 7.23 - 7.27 (m, 2H), 6.85 - 6.96 (m, 3H), 5.22 (s, 2H), 4.84 - 4.98 (m, 1H), 2.80 (dd, *J =* 5.04, 7.48 Hz, 2H), 2.32 - 2.43 (m, 1H), 1.65 (d, *J =* 6.71 Hz, 6H), 0.95 - 1.09 (m, 1H), 0.55 - 0.63 (m, 1H), 0.38 - 0.46 (m, 1H), 0.26 - 0.33 (m, 1H), 0.11 - 0.22 (m, 1H). MS (ESI): [M + H]⁺ = 455.11

### (11) Synthesis of 5-(4-((1-isopropyl-3-phenyl-1H-indazol-6-yl)methoxy)benzylidene)-2,2-dimethyl-1,3-dioxane-4,6-dione (A5)

The compound synthesized in (10) above (100 mg, 0.267 mmol) and Meldrum's acid (120 mg, 0.801 mmol) were dissolved in EtOH (4 mL), and piperidine (50 µL) and AcOH (50 µL) were added. The mixture was irradiated at 80°C for 30 min using a microwave reactor and concentrated under reduced pressure. The residue was then purified by silica gel column chromatography (hexane:ethyl acetate = 4:1) to obtain Compound as a white solid A5 (92 mg, 69% yield).

¹H NMR (CDCl₃, 400 MHz): δ 8.40 (s, 1H), 8.23 - 8.28 (m, 2H), 8.02 - 8.07 (m, 1H), 7.94 - 8.00 (m, 2H), 7.55 (s, 1H), 7.48 - 7.54 (m, 2H), 7.36 - 7.43 (m, 1H), 7.24 (dd, *J=* 1.37, 8.39 Hz, 1H), 7.10 - 7.14 (m, 2H), 5.34 (s, 2H), 4.91 (quin, *J =* 6.71 Hz, 1H), 1.80 (s, 6H), 1.66 (d, *J=* 6.71 Hz, 6H). MS (ESI): [M + H]⁺ = 497.3

### (12) Synthesis of 3-(4-((1-isopropyl-3-phenyl-1H-indazol-6-yl)methoxy)phenyl)butanoic acid (Compound 7)

A5 (20 mg, 0.040 mmol) was dissolved in THF (2 mL). The reaction mixture was cooled to 0°C, and a 3.0 M THF solution of methylmagnesium bromide (16 µL, 0.048 mmol) was added dropwise and the mixture was stirred for 30 min. After the reaction was complete, DMF (9 mL) and H₂O (1 mL) were added, the mixture was heated to 100°C, and stirred for 1 h. The reaction mixture was poured into water and extracted with ethyl acetate. The combined extracts were dried over Na₂SO₄, filtered, and concentrated under reduced pressure to yield a crude product. The crude product was then purified by silica gel column chromatography (CHCl₃:MeOH = 25:1) to obtain Compound 7 as a yellow solid (7.4 mg, 43% yield).

¹H NMR (CDCl₃, 400 MHz): δ 7.95 - 8.05 (m, 3H), 7.55 (s, 1H), 7.47 - 7.53 (m, 2H), 7.37 - 7.42 (m, 1H), 7.24 (dd, *J =* 1.22, 8.24 Hz, 1H), 7.14 - 7.21 (m, 2H), 6.95 - 7.02 (m, 2H), 5.21 (s, 2H), 4.90 (quin, *J =* 6.64 Hz, 1H), 3.22 - 3.34 (m, 1H), 2.52 - 2.70 (m, 2H), 1.65 (d, *J=* 6.71 Hz, 6H), 1.32 (d, *J=* 7.02 Hz, 3H). MS (ESI): [M + H]⁺ = 429.01

### (13) Synthesis of 3-(4-((1-isopropyl-3-phenyl-1H-indazol-6-yl)methoxy)phenyl)-4-methylpentanoic acid (Compound 8)

Compound 8 as a yellow solid (32% yield) was obtained using A5 (20 mg, 0.040 mmol) and a THF solution of isopropylmagnesium bromide in the same manner as described in (12) above.

¹H NMR (CDCl₃, 400 MHz): δ 8.01 (dd, *J=* 0.61, 8.55 Hz, 1H), 7.95 - 7.99 (m, 2H), 7.55 (s, 1H), 7.47 - 7.53 (m, 2H), 7.36 - 7.41 (m, 1H), 7.24 (dd, *J =* 1.22, 8.24 Hz, 1H), 7.07 - 7.13 (m, 2H), 6.93 - 6.98 (m, 2H), 5.20 (s, 2H), 4.83 - 4.95 (m, 1H), 2.76 - 2.88 (m, 2H), 2.56 - 2.66 (m, 1H), 1.78 - 1.88 (m, 1H), 1.64 (d, *J =* 6.71 Hz, 6H), 0.94 (d, *J =* 6.71 Hz, 3H), 0.77 (d, *J=* 6.71 Hz, 3H)). MS (ESI): [M + H]⁺ = 457.07

### (14) Synthesis of 3-(4-((1-isopropyl-3-phenyl-1H-indazol-6-yl)methoxy)phenyl)pentanoic acid (Compound 9)

Compound 9 as a yellow solid (31% yield) was obtained using A5 (20 mg, 0.040 mmol) and a THF solution of ethylmagnesium bromide in the same manner as described in (12) above.

### Preparative Example 2. Synthesis of Compounds 10 to 34

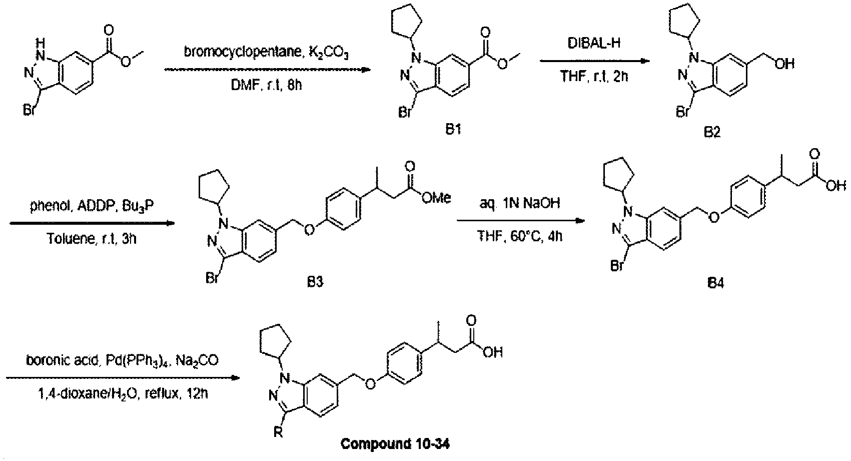

### (1) Synthesis of methyl 3-bromo-1-cyclopentyl-1H-indazole-6-carboxylate (Compound B1)

Methyl 3-bromo-1H-indazole-6-carboxylate (20 mg, 0.078 mmol) and K₂CO₃ (32 mg, 0.234 mmol) were dissolved in DMF (0.4 mL), and bromocyclopentane (13 µL, 0.117 mmol) was added dropwise to the solution. The reaction mixture was stirred at room temperature for 8 h, poured into water, and extracted with ethyl acetate. The combined extracts were dried over Na₂SO₄, filtered, and concentrated under reduced pressure to yield a crude product. The crude product was purified by silica gel column chromatography (hexane:ethyl acetate = 6:1) to obtain Compound B1 as a colorless oil (24.6 mg, 98% yield).

¹H NMR (CDCl₃, 400 MHz): δ 8.22 (t, *J=* 1.07 Hz, 1H), 7.85 (dd, *J* = 1.22, 8.55 Hz, 1H), 7.64 (dd, *J=* 0.61, 8.55 Hz, 1H), 5.04 (quin, *J* = 7.32 Hz, 1H), 3.99 (s, 3H), 2.15 - 2.23 (m, 4H), 1.95 - 2.05 (m, 2H), 1.69 - 1.83 (m, 2H). MS (ESI): [M + H]⁺ = 323.3

### (2) Synthesis of (3-bromo-1-cyclopentyl-1H-indazol-6-yl)methanol (Compound B2)

B1 (20 mg, 0.062 mmol) was dissolved in THF (0.6 mL). A 1 M DIBAL-H solution in toluene (160 µL, 0.160 mmol) was added dropwise at 0°C. The reaction mixture was stirred at room temperature for 2 h, poured into 1 N aqueous HCl solution, and extracted with ethyl acetate. The combined extracts were dried over Na₂SO₄, filtered, and concentrated under reduced pressure to yield a crude product. The crude product was purified by silica gel column chromatography (CHCl₃:MeOH = 300:1) to obtain Compound B2 as a colorless oil.

¹H NMR (CDCl₃, 400 MHz): δ 7.54 - 7.59 (m, 1H), 7.48 (d, J = 0.92 Hz, 1H), 7.11 - 7.19 (m, 1H), 4.96 (quin, J = 7.48 Hz, 1H), 4.88 (d, J = 5.19 Hz, 2H), 2.12 - 2.22 (m, 4H), 1.90 - 2.06 (m, 2H), 1.66 - 1.79 (m, 2H). MS (ESI): [M + H]+ = 297.00.

### (3) Synthesis of methyl 3-(4-((3-bromo-1-cyclopentyl-1H-indazol-6-yl)methoxy)phenyl)butanoate (Compound B3)

B2 (10 mg, 0.034 mmol) was dissolved in toluene (2 mL), and methyl 3-(4-hydroxyphenyl)butanoate (9.9 mg, 0.051 mmol) was added. Subsequently, tributylphosphine (25 µL, 0.102 mmol) and 1,1'-(azodicarbonyl)dipiperidine (26 mg, 0.102 mmol) were added, and the mixture was stirred at room temperature for 3 h. The reaction mixture was then filtered and washed with hexane. The filtrate was then concentrated under reduced pressure to yield a crude product. The crude product was purified by silica gel column chromatography (hexane:ethyl acetate = 8:1) to obtain Compound B3 as a colorless oil (11 mg, 69% yield).

¹H NMR (CDCl₃, 400 MHz): δ 7.60 (dd, *J* = 0.90, 8.60 Hz, 1H), 7.50 - 7.53 (m, 1H), 7.22 (dd, *J =* 1.22, 8.24 Hz, 1H), 7.12 - 7.20 (m, 2H), 6.92 - 6.97 (m, 2H), 5.19 (s, 2H), 4.90 - 5.02 (m, 1H), 3.63 (s, 3H), 3.19 - 3.30 (m, 1H), 2.48 - 2.66 (m, 2H), 2.13 - 2.22 (m, 4H), 1.94 - 2.04 (m, 2H), 1.71 - 1.83 (m, 2H), 1.29 (d, *J=* 6.71 Hz, 3H). MS (ESI): [M + H]⁺ = 471.1

### (4) Synthesis of 3-(4-((3-bromo-1-cyclopentyl-1H-indazol-6-yl)methoxy)phenyl)butanoic acid (Compound B4).

B3 (10 mg, 0.0212 mmol) was dissolved in THF (0.3 mL). 1 N aqueous NaOH solution (0.1 mL) was added dropwise, and the mixture was stirred at 60°C for 4 h. The reaction mixture was then poured into 1 N aqueous HCl solution and extracted with ethyl acetate. The combined extracts were dried over Na₂SO₄, filtered, and concentrated under reduced pressure to yield a crude product. The crude product was purified by silica gel column chromatography (CHCl₃:MeOH = 20:1) to obtain Compound B4 as a white solid (8.2 mg, 85% yield).

¹H NMR (CDCl₃, 400 MHz): δ 7.59 (d, *J* = 11.00 Hz, 1H), 7.52 (s, 1H), 7.22 (dd, *J=* 1.15, 8.41 Hz, 1H), 7.14 - 7.20 (m, 2H), 6.92 - 6.99 (m, 2H), 5.19 (s, 2H), 4.88 - 5.03 (m, 1H), 3.26 (d, *J =* 7.25 Hz, 1H), 2.52 - 2.71 (m, 2H), 2.10 - 2.25 (m, 4H), 1.98 (d, *J* = 3.30 Hz, 2H), 1.70 - 1.77 (m, 2H), 1.31 (d, *J =* 6.92 Hz, 3H). MS (ESI): [M + H]⁺ = 458.93

### (5) Synthesis of 3-(4-((1-cyclopentyl-3-(o-tolyl)-1H-indazol-6-yl)methoxy)phenyl)butanoic acid (Compound 10)

B4 (20 mg, 0.043 mmol), Na₂CO₃ (14 mg, 0.131 mmol), o-tolylboronic acid (9 mg, 0.064 mmol), and Pd(PPh3)4 (10 mg, 0.008 mmol) were added to 1,4-dioxane (2 mL) and water (1 mL), and stirred under reflux for 12 h. The reaction mixture was then evaporated under reduced pressure. The residue was taken up in water and extracted with ethyl acetate. The combined extracts were dried over Na₂SO₄, filtered, and concentrated under reduced pressure to yield a crude product. The crude product was purified by silica gel column chromatography (CHCl₃:MeOH = 50:1) to obtain Compound 10 as a white solid (8 mg, 40% yield).

¹H NMR (CDCl₃, 400 MHz): δ 7.68 (d, *J* = 8.24 Hz, 1H), 7.58 (s, 1H), 7.53 - 7.57 (m, 1H), 7.33 - 7.37 (m, 1H), 7.29 - 7.33 (m, 2H), 7.16 - 7.21 (m, 3H), 6.96 - 7.00 (m, 2H), 5.21 (s, 2H), 5.05 (quin, *J =* 7.17 Hz, 1H), 3.22 - 3.30 (m, 1H), 2.53 - 2.70 (m, 2H), 2.43 (s, 3H), 2.17 - 2.29 (m, 4H), 1.97 - 2.05 (m, 2H), 1.72 - 1.79 (m, 2H), 1.32 (d, *J* = 7.02 Hz, 3H). MS (ESI): [M + H]⁺ = 469.08

### (6) Synthesis of 3-(4-((1-cyclopentyl-3-(p-tolyl)-1H-indazol-6-yl)methoxy)phenyl)butanoic acid (Compound 11)

Compound 11 was synthesized from B4 and p-tolylboronic acid in the same manner as described for the synthesis of Compound 10, to afford a white solid (67% yield).

¹H NMR (CDCl₃, 400 MHz): δ 7.99 (d, *J =* 8.24 Hz, 1H), 7.84 - 7.89 (m, 2H), 7.55 (s, 1H), 7.28 - 7.34 (m, *J =* 7.93 Hz, 2H), 7.22 (dd, *J=* 1.22, 8.24 Hz, 1H), 7.16 - 7.19 (m, 2H), 6.96 - 7.01 (m, 2H), 5.21 (s, 2H), 5.00 - 5.07 (m, 1H), 3.21 - 3.29 (m, 1H), 2.54 - 2.65 (m, 2H), 2.42 (s, 3H), 2.14 - 2.28 (m, 4H), 1.96 - 2.04 (m, 2H), 1.73 - 1.79 (m, 2H), 1.31 (d, *J=* 7.02 Hz, 3H). MS (ESI): [M + H]⁺ =469.08

### (7) Synthesis of 3-(4-((1-cyclopentyl-3-(4-hydroxyphenyl)-1H-indazol-6-yl)methoxy)phenyl)butanoic acid (Compound 12)

Compound 12 was synthesized from B4 and (4-hydroxyphenyl)boronic acid in the same manner as described for the synthesis of Compound 10, to afford a yellow solid (47% yield).

¹H NMR (CDCl₃, 400 MHz): δ 7.95 (d, *J =* 8.55 Hz, 1H), 7.83 - 7.87 (m, 2H), 7.53 (s, 1H), 7.21 (dd, *J =* 1.22, 8.55 Hz, 1H), 7.16 - 7.20 (m, 2H), 6.94 - 6.99 (m, 4H), 5.20 (s, 2H), 4.96 - 5.06 (m, 1H), 3.21 - 3.30 (m, 1H), 2.54 - 2.69 (m, 2H), 2.18 - 2.28 (m, 4H), 1.97 - 2.05 (m, 2H), 1.73 - 1.78 (m, 2H), 1.31 (d, *J =* 5.80 Hz, 3H). MS (ESI): [M + H]⁺ =471.03

### (8) Synthesis of 3-(4-((1-cyclopentyl-3-(2-hydroxyphenyl)-1H-indazol-6-yl)methoxy)phenyl)butanoic acid (Compound 13)

Compound 13 was synthesized from B4 and (2-hydroxyphenyl)boronic acid in the same manner as described for the synthesis of Compound 10, to afford a yellow solid (40% yield).

¹H NMR (CDCl₃, 400 MHz): δ 8.21 (d, *J* = 8.55 Hz, 1H), 8.07 (dd, *J* = 1.53, 7.94 Hz, 1H), 7.61 (s, 1H), 7.27 - 7.32 (m, 2H), 7.16 - 7.22 (m, 2H), 7.10 - 7.13 (m, 1H), 7.02 - 7.06 (m, 1H), 6.94 - 7.00 (m, 2H), 5.23 (s, 2H), 5.03 - 5.11 (m, 1H), 3.19 - 3.29 (m, 1H), 2.55 - 2.72 (m, 2H), 2.16 - 2.24 (m, 4H), 2.01 (dt, *J=* 1.07, 3.13 Hz, 2H), 1.78 - 1.83 (m, 2H), 1.32 (d, *J=* 7.02 Hz, 3H). MS (ESI): [M + H]⁺ = 471.3

### (9) Synthesis of 3-(4-((1-cyclopentyl-3-(3-hydroxyphenyl)-1H-indazol-6-yl)methoxy)phenyl)butanoic acid (Compound 14)

Compound 14 was synthesized from B4 and (3-hydroxyphenyl)boronic acid in the same manner as described for the synthesis of Compound 10, to afford a yellow solid (33% yield).

¹H NMR (CDCl₃, 400 MHz): δ 8.20 (d, *J =* 8.55 Hz, 1H), 8.07 (dd, *J=* 1.53, 7.93 Hz, 1H), 7.61 (s, 1H), 7.27 - 7.33 (m, 2H), 7.16 - 7.22 (m, 2H), 7.10 - 7.14 (m, 1H), 7.02 - 7.06 (m, 1H), 6.96 - 7.00 (m, 2H), 5.22 (s, 2H), 5.05 - 5.11 (m, 1H), 3.22 - 3.30 (m, 1H), 2.57 - 2.67 (m, 2H), 2.16 - 2.23 (m, 4H), 1.98 - 2.03 (m, 2H), 1.77 - 1.83 (m, 2H), 1.32 (d, *J= 7.02* Hz, 3H). MS (ESI): [M - H]⁻ = 469.5

### (10) Synthesis of 3-(4-((1-cyclopentyl-3-(4-methoxyphenyl)-1H-indazol-6-yl)methoxy)phenyl)butanoic acid (Compound 15)

Compound 15 was synthesized from B4 and (4-methoxyphenyl)boronic acid in the same manner as described for the synthesis of Compound 10, to afford a white solid (62% yield).

¹H NMR (CDCl₃, 400 MHz): δ 7.97 (d, *J =* 8.55 Hz, 1H), 7.87 - 7.92 (m, 2H), 7.54 (s, 1H), 7.21 (dd, *J =* 1.22, 8.55 Hz, 1H), 7.15 - 7.20 (m, 2H), 7.02 - 7.06 (m, 2H), 6.95 - 7.00 (m, 2H), 5.20 (s, 2H), 4.97 - 5.05 (m, 1H), 3.88 (s, 3H), 3.14 - 3.35 (m, 1H), 2.52 - 2.72 (m, 2H), 2.13 - 2.30 (m, 4H), 1.98 - 2.06 (m, 2H), 1.74 - 1.79 (m, 2H), 1.31 (d, *J= 6.71* Hz, 3H). MS (ESI): [M + H]⁺ = 485.07

### (11) Synthesis of 3-(4-((1-cyclopentyl-3-(4-nitrophenyl)-1H-indazol-6-yl)methoxy)phenyl)butanoic acid (Compound 16)

Compound 16 was synthesized from B4 and (4-nitrophenyl)boronic acid in the same manner as described for the synthesis of Compound 10, to afford a yellow solid (41% yield).

¹H NMR (CDCl₃, 400 MHz): δ 8.33 - 8.38 (m, 2H), 8.16 - 8.21 (m, 2H), 8.03 (d, *J* = 8.47 Hz, 1H), 7.62 (s, 1H), 7.32 (dd, *J =* 1.37, 8.24 Hz, 1H), 7.15 - 7.23 (m, 2H), 6.95 - 7.05 (m, 2H), 5.23 (s, 2H), 5.04 - 5.14 (m, 1H), 3.20 - 3.28 (m, 1H), 2.53 - 2.69 (m, 2H), 2.16 - 2.32 (m, 4H), 1.98 - 2.07 (m, 2H), 1.76 - 1.85 (m, 2H), 1.31 (d, *J =* 6.87 Hz, 3H). MS (ESI): [M + H]⁺ =500.05

### (12) Synthesis of 3-(4-((1-cyclopentyl-3-(4-(trifluoromethyl)phenyl)-1H-indazol-6-yl)methoxy)phenyl)butanoic acid (Compound 17)

Compound 17 was synthesized from B4 and (4-(trifluoromethyl)phenyl)boronic acid in the same manner as described for the synthesis of Compound 10, to afford a white solid (42% yield).

¹H NMR (CDCl₃, 400 MHz): δ 8.08 - 8.16 (m, 2H), 8.00 (d, *J =* 8.24 Hz, 1H), 7.71 - 7.79 (m, 2H), 7.59 - 7.63 (m, 1H), 7.29 - 7.33 (m, 1H), 7.14 - 7.24 (m, 2H), 6.95 - 7.03 (m, 2H), 5.24 (s, 2H), 5.01 - 5.13 (m, 1H), 3.22 - 3.30 (m, 1H), 2.54 - 2.69 (m, 2H), 2.17 - 2.30 (m, 4H), 2.00 - 2.09 (m, 2H), 1.74 - 1.84 (m, 2H), 1.32 (d, *J=* 6.71 Hz, 3H). MS (ESI): [M + H]⁺ = 523.5.

### (13) Synthesis of 3-(4-((3-(4-chlorophenyl)-1-cyclopentyl-1H-indazol-6-yl)methoxy)phenyl)butanoic acid (Compound 18)

Compound 18 was synthesized from B4 and (4-chlorophenyl)boronic acid in the same manner as described for the synthesis of Compound 10, to afford a white solid (33% yield).

¹H NMR (CDCl₃, 400 MHz): δ 7.95 (d, *J =* 7.32 Hz, 1H), 7.89 - 7.93 (m, 2H), 7.57 (s, 1H), 7.42 - 7.49 (m, 2H), 7.24 (dd, *J =* 1.22, 8.55 Hz, 1H), 7.14 - 7.20 (m, 2H), 6.94 - 7.00 (m, 2H), 5.18 - 5.22 (m, 2H), 4.99 - 5.08 (m, 1H), 3.21 - 3.30 (m, 1H), 2.52 - 2.66 (m, 2H), 2.16 - 2.30 (m, 4H), 1.96 - 2.07 (m, 2H), 1.72 - 1.87 (m, 2H), 1.31 (d, *J=* 7.02 Hz, 3H). MS (ESI): [M + H]⁺ = 489.2

### (14) Synthesis of 3-(4-((3-(4-cyanophenyl)-1-cyclopentyl-1H-indazol-6-yl)methoxy)phenyl)butanoic acid (Compound 19)

Compound 19 was synthesized from B4 and (4-cyanophenyl)boronic acid in the same manner as described for the synthesis of Compound 10, to afford a white solid (42% yield).

¹H NMR (CDCl₃, 400 MHz): δ 8.09 - 8.14 (m, 2H), 7.99 (d, *J* = 8.24 Hz, 1H), 7.74 - 7.79 (m, 2H), 7.60 (s, 1H), 7.29 (dd, *J =* 1.22, 8.55 Hz, 1H), 7.16 - 7.21 (m, 2H), 6.95 - 7.00 (m, 2H), 5.22 (s, 2H), 5.01 - 5.10 (m, 1H), 3.21 - 3.31 (m, 1H), 2.55 - 2.67 (m, 2H), 2.18 - 2.27 (m, 4H), 1.99 - 2.05 (m, 2H), 1.75 - 1.81 (m, 2H), 1.32 (d, *J* = 7.02 Hz, 3H). MS (ESI): [M + H]⁺ = 480.03

### (15) Synthesis of 3-(4-((1-cyclopentyl-3-(3,5-dichloro-4-hydroxyphenyl)-1H-indazol-6-yl)methoxy)phenyl)butanoic acid (Compound 20)

Compound 20 was synthesized from B4 and (3,5-dichloro-4-hydroxyphenyl)boronic acid in the same manner as described for the synthesis of Compound 10, to afford a white solid (46% yield).

¹H NMR (CDCl₃, 400 MHz): δ 7.93 (d, *J =* 8.70 Hz, 1H), 7.89 (s, 2H), 7.57 (s, 1H), 7.25 (d, *J=* 0.92 Hz, 1H), 7.13 - 7.20 (m, 2H), 6.93 - 7.01 (m, 2H), 5.21 (s, 2H), 5.02 (quin, *J* = 7.33 Hz, 1H), 3.21 - 3.29 (m, 1H), 2.55 - 2.68 (m, 2H), 2.16 - 2.26 (m, 4H), 2.02 (dt, *J=* 2.06, 7.67 Hz, 2H), 1.74 - 1.81 (m, 2H), 1.32 (d, *J =* 7.33 Hz, 3H). MS (ESI): [M + H]⁺ = 539.5

### (16) Synthesis of 3-(4-((1-cyclopentyl-3-(3,5-dichloro-4-(hydroxymethyl)phenyl)-1H-indazol-6-yl)methoxy)phenyl)butanoic acid (Compound 21)

Compound 21 was synthesized from B4 and (3,5-dichloro-4-(hydroxymethyl)phenyl)boronic acid in the same manner as described for the synthesis of Compound 10, to afford a white solid (41% yield).

¹H NMR (CDCl₃, 400 MHz): δ 7.90 - 7.99 (m, 3H), 7.59 (s, 1H), 7.30 (d, *J* = 0.92 Hz, 1H), 7.15 - 7.21 (m, 2H), 6.94 - 7.00 (m, 2H), 5.21 (s, 2H), 5.00 - 5.08 (m, 3H), 3.19 - 3.30 (m, 1H), 2.54 - 2.68 (m, 2H), 2.15 - 2.28 (m, 4H), 1.98 - 2.06 (m, 2H), 1.73 - 1.80 (m, 2H), 1.32 (d, *J=* 7.33 Hz, 3H). MS (ESI): [M + H]⁺ = 554.65

### (17) Synthesis of 3-(4-((3-(3,5-bis(trifluoromethyl)phenyl)-1-cyclopentyl-1H-indazol-6-yl)methoxy)phenyl)butanoic acid (Compound 22)

Compound 22 was synthesized from B4 and (3,5-bis(trifluoromethyl)phenyl)boronic acid in the same manner as described for the synthesis of Compound 10, to afford a white solid (21% yield).

### (18) Synthesis of 3-(4-((1-cyclopentyl-3-(pyridin-4-yl)-1H-indazol-6-yl)methoxy)phenyl)butanoic acid (Compound 23)

Compound 23 was synthesized from B4 and pyridin-4-ylboronic acid in the same manner as described for the synthesis of Compound 10, to afford a white solid (27% yield).

### (19) Synthesis of 3-(4-((3-([1,1'-biphenyl]-4-yl)-1-cyclopentyl-1H-indazol-6-yl)methoxy)phenyl)butanoic acid (Compound 24)

Compound 24 was synthesized from B4 and [1,1'-biphenyl]-4-ylboronic acid in the same manner as described for the synthesis of Compound 10, to afford a white solid (17% yield).

### (20) Synthesis of 3-(4-((3-(benzo[b]thiophen-2-yl)-1-cyclopentyl-1H-indazol-6-yl)methoxy)phenyl)butanoic acid (Compound 25)

Compound 25 was synthesized from B4 and benzo[b]thiophen-2-ylboronic acid in the same manner as described for the synthesis of Compound 10, to afford a white solid (22% yield).

### (21) Synthesis of 3-(4-((1-cyclopentyl-3-(naphthalen-1-yl)-1H-indazol-6-yl)methoxy)phenyl)butanoic acid (Compound 26)

Compound 26 was synthesized from B4 and naphthalen-1-ylboronic acid in the same manner as described for the synthesis of Compound 10, to afford a white solid (31% yield).

### (22) Synthesis of 3-(4-((1-cyclopentyl-3-(4-(pentan-3-yloxy)phenyl)-1H-indazol-6-yl)methoxy)phenyl)butanoic acid (Compound 27)

Compound 27 was synthesized from B4 and (4-(pentan-3-yloxy)phenyl)boronic acid in the same manner as described for the synthesis of Compound 10, to afford a white solid (20% yield).

### (23) Synthesis of (E)-4-(4-(6-((4-(1-carboxypropan-2-yl)phenoxy)methyl)-1-cyclopentyl-1H-indazol-3-yl)phenoxy)but-2-enoic acid (Compound 28)

Compound 28 was synthesized from B4 and (E)-4-(4-boronophenoxy)but-2-enoic acid in the same manner as described for the synthesis of Compound 10, to afford a white solid (19% yield).

### (24) Synthesis of 3-(4-((1-cyclopentyl-3-(4-ethoxyphenyl)-1H-indazol-6-yl)methoxy)phenyl)butanoic acid (Compound 29)

Compound 29 was synthesized from B4 and (4-ethoxyphenyl)boronic acid in the same manner as described for the synthesis of Compound 10, to afford a white solid (38% yield).

### (25) Synthesis of 3-(4-((1-cyclopentyl-3-(4-ethoxyphenyl)-1H-indazol-6-yl)methoxy)phenyl)butanoic acid (Compound 29)

Compound 29 was synthesized from B4 and (4-ethoxyphenyl)boronic acid in the same manner as described for the synthesis of Compound 10, to afford a white solid (38% yield).

### (26) Synthesis of 3-(4-((3-(4-(allyloxy)phenyl)-1-cyclopentyl-1H-indazol-6-yl)methoxy)phenyl)butanoic acid (Compound 30)

Compound 30 was synthesized from B4 and (4-(allyloxy)phenyl)boronic acid in the same manner as described for the synthesis of Compound 10, to afford a white solid (23% yield).

### (27) Synthesis of 3-(4-((3-(Benzo[d][1,3]dioxol-5-yl)-1-cyclopentyl-1H-indazol-6-yl)methoxy)phenyl)butanoic acid (Compound 31)

Compound 31 was synthesized from B4 and benzo[d][1,3]dioxol-5-ylboronic acid in the same manner as described for the synthesis of Compound 10, to afford a white solid (31% yield).

### (28) Synthesis of 3-(4-((1-cyclopentyl-3-(1H-tetrazol-5-yl)-1H-indazol-6-yl)methoxy)phenyl)butanoic acid (Compound 32)

Compound 32 was synthesized from B4 and (1H-tetrazol-5-yl)boronic acid in the same manner as described for the synthesis of Compound 10, to afford a white solid (19% yield).

### (29) Synthesis of 3-(4-((1-cyclopentyl-3-(2,6-dichloropyridin-4-yl)-1H-indazol-6-yl)methoxy)phenyl)butanoic acid (Compound 33)

Compound 33 was synthesized from B4 and (2,6-dichloropyridin-4-yl)boronic acid in the same manner as described for the synthesis of Compound 10, to afford a white solid (31% yield).

### (30) Synthesis of (E)-3-(4-((1-cyclopentyl-3-(3,5-difluoro-4-((hydroxyimino)methyl)phenyl)-1H-indazol-6-yl)methoxy)phenyl)butanoic acid (Compound 34)

Compound 34 was synthesized from B4 and (E)-(3,5-difluoro-4-((hydroxyimino)methyl)phenyl)boronic acid in the same manner as described for the synthesis of Compound 10, to afford a white solid (10% yield).

### Preparative Example 3. Synthesis of Compounds 35 to 55

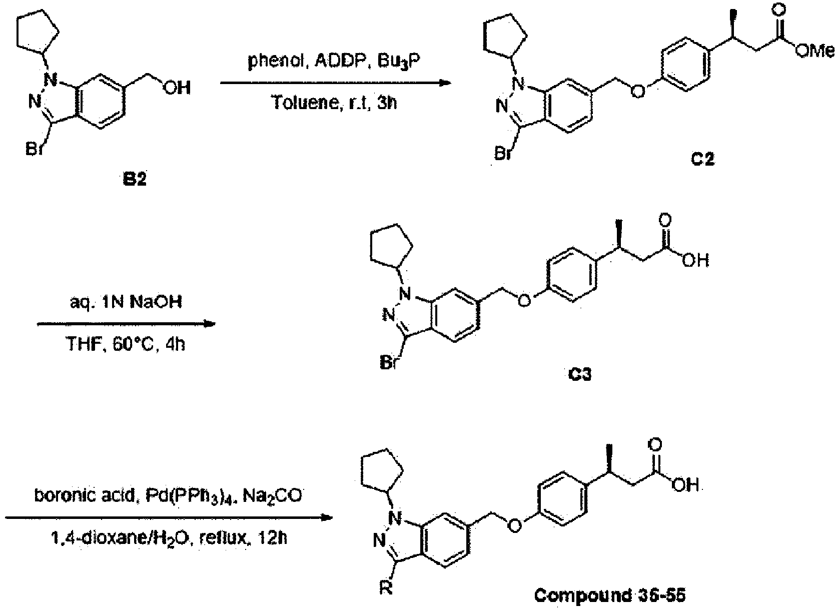

### (1) Synthesis of methyl (S)-3-(4-((3-bromo-1-cyclopentyl-1H-indazol-6-yl)methoxy)phenyl)butanoate (C2)

B2 (0.034 mmol) was dissolved in toluene (2 mL), and methyl (S)-3-(4-hydroxyphenyl)butanoate (0.051 mmol) was added. Subsequently, tributylphosphine (25 µL, 0.102 mmol) and 1,1'-(azodicarbonyl)dipiperidine (26 mg, 0.102 mmol) were added, and the mixture was stirred at room temperature for 3 h. The reaction mixture was then filtered and washed with hexane. The filtrate was then concentrated under reduced pressure to yield a crude product. The crude product was purified by silica gel column chromatography (hexane:ethyl acetate = 8:1) to obtain Compound C2 as a colorless oil (57% yield).

¹H NMR (CDCl₃, 400 MHz): δ 7.60 (dd, *J* = 0.61, 8.24 Hz, 1H), 7.52 (s, 1H), 7.22 (dd, *J =* 1.22, 8.24 Hz, 1H), 7.14 - 7.18 (m, 2H), 6.93 - 6.97 (m, 2H), 5.19 (s, 2H), 4.91 - 5.01 (m, 1H), 3.63 (s, 3H), 3.21 - 3.30 (m, 1H), 2.50 - 2.64 (m, 2H), 2.12 - 2.20 (m, 4H), 1.93 - 2.04 (m, 2H), 1.70 - 1.79 (m, 2H), 1.29 (d, *J=* 7.02 Hz, 3H). MS (ESI): [M + H]⁺ = 471.1

### (2) Synthesis of (S)-3-(4-((3-bromo-1-cyclopentyl-1H-indazol-6-yl)methoxy)phenyl)butanoic acid (C3)

C2 (0.034 mmol) was dissolved in toluene (2 mL), and methyl (S)-3-(4-hydroxyphenyl)butanoate (0.051 mmol) was added. Subsequently, tributylphosphine (25 µL, 0.102 mmol) and 1,1'-(azodicarbonyl)dipiperidine (26 mg, 0.102 mmol) were added, and the mixture was stirred at room temperature for 3 h. The reaction mixture was then filtered and washed with hexane. The filtrate was then concentrated under reduced pressure to yield a crude product. The crude product was purified by silica gel column chromatography (hexane: ethyl acetate = 8:1) to obtain Compound C3 as a white solid (82% yield).

¹H NMR (CDCl₃, 400 MHz): δ 7.60 (dd, *J* = 0.61, 8.55 Hz, 1H), 7.52 (s, 1H), 7.20 - 7.24 (m, 1H), 7.16 - 7.20 (m, 2H), 6.95 - 6.97 (m, 2H), 5.19 (s, 2H), 4.93 - 4.99 (m, 1H), 3.24 - 3.28 (m, 1H), 2.57 - 2.65 (m, 2H), 2.14 - 2.20 (m, 4H), 1.95 - 2.00 (m, 2H), 1.73 - 1.77 (m, 2H), 1.32 (d, *J=* 7.93 Hz, 3H). MS (ESI): [M + H]⁺ = 458.93

### (3) Synthesis of (S)-3-(4-((1-cyclopentyl-3-(4-hydroxy-3-(trifluoromethyl)phenyl)-1H-indazol-6-yl)methoxy)phenyl)butanoic acid (Compound 35)

Compound 35 was synthesized from C3 and (4-hydroxy-3-(trifluoromethyl)phenyl)boronic acid in the same manner as described for the synthesis of Compound 10, to afford a white solid (53% yield).

¹H NMR (CDCl₃, 400 MHz): δ 8.11 (d, *J =* 1.83 Hz, 1H), 7.99 - 8.04 (m, 1H), 7.89 - 7.95 (m, 1H), 7.57 (s, 1H), 7.23 - 7.26 (m, 1H), 7.15 - 7.20 (m, 2H), 7.09 (d, *J =* 7.93 Hz, 1H), 6.95 - 7.00 (m, 2H), 5.21 (s, 2H), 4.99 - 5.08 (m, 1H), 3.23 - 3.29 (m, 1H), 2.56 - 2.66 (m, 2H), 2.18 - 2.27 (m, 4H), 2.00 - 2.05 (m, 2H), 1.74 - 1.80 (m, 2H), 1.32 (d, *J= 7.02* Hz, 3H). MS (ESI): [M + H]⁺ 538.62

### (4) Synthesis of (S)-3-(4-((3-(3-chloro-4-hydroxyphenyl)-1-cyclopentyl-1H-indazol-6-yl)methoxy)phenyl)butanoic acid (Compound 36)

Compound 36 was synthesized from C3 and (3-chloro-4-hydroxyphenyl)boronic acid in the same manner as described for the synthesis of Compound 10, to afford a white solid (34% yield).

¹H NMR (CDCl₃, 400 MHz): δ 7.92 - 7.96 (m, 2H), 7.79 (dd, *J* = 1.98, 8.39 Hz, 1H), 7.56 (s, 1H), 7.23 (dd, *J =* 1.07, 8.39 Hz, 1H), 7.16 - 7.20 (m, 2H), 7.14 (d, *J=* 8.55 Hz, 1H), 6.95 - 7.00 (m, 2H), 5.20 (s, 2H), 4.97 - 5.07 (m, 1H), 3.22 - 3.30 (m, 1H), 2.53 - 2.69 (m, 2H), 2.18 - 2.32 (m, 4H), 2.00 - 2.06 (m, 2H), 1.72 - 1.83 (m, 2H), 1.31 (d, *J* = 7.02 Hz, 3H). MS (ESI): [M + H]⁺ = 504.64

### (5) Synthesis of (S)-3-(4-((1-cyclopentyl-3-(4-hydroxy-3-nitrophenyl)-1H-indazol-6-yl)methoxy)phenyl)butanoic acid (Compound 37)

Compound 37 was synthesized from C3 and (4-hydroxy-3-nitrophenyl)boronic acid in the same manner as described for the synthesis of Compound 10, to afford a white solid (52% yield).

¹H NMR (CDCl₃, 400 MHz): δ 8.69 (d, *J* = 2.14 Hz, 1H), 8.27 (dd, *J* = 2.14, 8.55 Hz, 1H), 7.97 (d, *J =* 8.55 Hz, 1H), 7.59 (s, 1H), 7.28 - 7.32 (m, 2H), 7.14 - 7.22 (m, *J =* 8.55 Hz, 2H), 6.92 - 7.03 (m, 2H), 5.22 (s, 2H), 4.98 - 5.07 (m, 1H), 3.21 - 3.33 (m, 1H), 2.51 - 2.74 (m, 2H), 2.14 - 2.30 (m, 4H), 1.70 - 1.85 (m, 2H), 1.56 - 1.69 (m, 2H), 1.32 (d, *J= 7.02* Hz, 3H). MS (ESI): [M + H]⁺ = 516.3

### (6) Synthesis of ((S)-3-(4-((1-cyclopentyl-3-(3,4-dihydroxyphenyl)-1H-indazol-6-yl)methoxy)phenyl)butanoic acid (Compound 38)

Compound 38 was synthesized from C3 and (3,4-dihydroxyphenyl)boronic acid in the same manner as described for the synthesis of Compound 10, to afford a white solid (33% yield).

¹H NMR (CDCl₃, 400 MHz): δ 7.88 (d, *J=* 8.41 Hz, 1H), 7.52 (s, 1H), 7.44 (d, *J* = 1.98 Hz, 1H), 7.36 (dd, *J=* 1.98, 8.24 Hz, 1H), 7.17 (d, *J =* 8.74 Hz, 3H), 6.95 (dd, *J =* 1.32, 8.41 Hz, 3H), 5.18 (s, 2H), 4.95 - 5.05 (m, 1H), 3.18 - 3.28 (m, 1H), 2.57 - 2.65 (m, 2H), 2.15 - 2.25 (m, 4H), 1.94 - 2.02 (m, 2H), 1.69 - 1.78 (m, 2H), 1.31 (d, *J* = 6.92 Hz, 3H). MS (ESI): [M + H]⁺ = 487.3

### (7) Synthesis of (S)-3-(4-((1-cyclopentyl-3-(4-hydroxy-3-methoxyphenyl)-1H-indazol-6-yl)methoxy)phenyl)butanoic acid (Compound 39)

Compound 39 was synthesized from C3 and (4-hydroxy-3-methoxyphenyl)boronic acid in the same manner as described for the synthesis of Compound 10, to afford a white solid (29% yield).

¹H NMR (CDCl₃, 400 MHz): δ 7.92 - 7.99 (m, 1H), 7.51 - 7.56 (m, 1H), 7.43 - 7.49 (m, 2H), 7.20 - 7.23 (m, 1H), 7.18 (d, *J =* 8.85 Hz, 2H), 7.02 - 7.06 (m, 1H), 6.95 - 6.99 (m, 2H), 5.19 (s, 2H), 4.98 - 5.06 (m, 1H), 3.99 (s, 3H), 3.21 - 3.28 (m, 1H), 2.52 - 2.72 (m, 2H), 2.16 - 2.28 (m, 4H), 1.98 - 2.05 (m, 2H), 1.72 - 1.77 (m, 2H), 1.31 (d, *J=* 7.02 Hz, 3H). MS (ESI): [M + H]⁺ = 501.4

### (8) Synthesis of (S)-3-(4-((3-(3-cyano-4-hydroxyphenyl)-1-cyclopentyl-1H-indazol-6-yl)methoxy)phenyl)butanoic acid (Compound 40)

Compound 40 was synthesized from C3 and (3-cyano-4-hydroxyphenyl)boronic acid in the same manner as described for the synthesis of Compound 10, to afford a white solid (45% yield).

¹H NMR (CDCl₃, 400 MHz): δ 8.01 - 8.13 (m, 2H), 7.89 (d, *J* = 8.24 Hz, 1H), 7.57 (s, 1H), 7.24 (d, *J =* 8.55 Hz, 1H), 7.14 - 7.21 (m, *J =* 8.55 Hz, 2H), 7.07 (d, *J =* 8.85 Hz, 1H), 6.91 - 7.01 (m, 2H), 5.20 (s, 2H), 4.97 - 5.08 (m, 1H), 3.19 - 3.30 (m, 1H), 2.53 - 2.71 (m, 2H), 2.13 - 2.29 (m, 4H), 1.96 - 2.05 (m, 2H), 1.72 - 1.81 (m, 2H), 1.33 (d, *J* = 7.02 Hz, 3H) MS (ESI): [M + H]⁺ = 496.3

### (9) Synthesis of (S)-3-(4-((1-cyclopentyl-3-(2-fluoro-4-hydroxyphenyl)-1H-indazol-6yl)methoxy)phenyl)butanoic acid (Compound 41)

Compound 41 was synthesized from C3 and (2-fluoro-4-hydroxyphenyl)boronic acid in the same manner as described for the synthesis of Compound 10, to afford a white solid (30% yield).

¹H NMR (CDCl₃, 400 MHz): δ 7.78 (dd, *J* = 3.20, 8.39 Hz, 1H), 7.61 (t, *J* = 8.09 Hz, 1H), 7.52 - 7.56 (m, 1H), 7.15 - 7.22 (m, 3H), 6.94 - 7.01 (m, 2H), 6.66 - 6.73 (m, 2H), 5.20 (s, 2H), 5.04 (quin, *J* = 7.55 Hz, 1H), 3.19 - 3.30 (m, 1H), 2.54 - 2.70 (m, 2H), 2.14 - 2.30 (m, 4H), 1.94 - 2.05 (m, 2H), 1.67 - 1.82 (m, 2H), 1.32 (dd, *J* = 1.00, 6.71 Hz, 3H). MS (ESI): [M + H]⁺ = 488.67

### (10) Synthesis of (S)-3-(4-((3-(2-chloro-4-hydroxyphenyl)-1-cyclopentyl-1H-indazol-6-yl)methoxy)phenyl)butanoic acid (Compound 42)

Compound 42 was synthesized from C3 and (2-chloro-4-hydroxyphenyl)boronic acid in the same manner as described for the synthesis of Compound 10, to afford a white solid (52% yield).

¹H NMR (CDCl₃, 400 MHz): δ 7.66 (d, *J =* 8.55 Hz, 1H), 7.57 (s, 1H), 7.46 (d, *J* = 8.24 Hz, 1H), 7.16 - 7.21 (m, 3H), 7.04 (d, *J =* 2.44 Hz, 1H), 6.95 - 7.00 (m, 2H), 6.84 (dd, *J* = 2.59, 8.39 Hz, 1H), 5.21 (s, 2H), 5.00 - 5.10 (m, 1H), 3.20 - 3.30 (m, 1H), 2.52 - 2.70 (m, 2H), 2.15 - 2.32 (m, 4H), 1.95 - 2.03 (m, 2H), 1.72 - 1.79 (m, 2H), 1.31 (d, *J* = 7.02 Hz, 3H)) MS (ESI): [M + H]⁺ = 505.3

### (11) Synthesis of (S)-3-(4-((1-cyclopentyl-3-(3,5-difluoro-4-hydroxyphenyl)-1H-indazol-6-yl)methoxy)phenyl)butanoic acid (Compound 43)

Compound 43 was synthesized from C3 and (3,5-difluoro-4-hydroxyphenyl)boronic acid in the same manner as described for the synthesis of Compound 10, to afford a white solid (49% yield).

¹H NMR (METHANOL-d₄, 400 MHz): δ 7.89 (d, *J =* 8.47 Hz, 1H), 7.61 (s, 1H), 7.39 - 7.53 (m, 2H), 7.23 (dd, *J =* 1.15, 8.47 Hz, 1H), 7.08 - 7.18 (m, 2H), 6.86 - 6.97 (m, 2H), 5.15 (s, 2H), 5.07 (t, *J =* 7.21 Hz, 1H), 3.01 - 3.18 (m, 1H), 2.41 - 2.53 (m, 2H), 2.04 - 2.21 (m, 4H), 1.89 - 2.03 (m, 2H), 1.67 - 1.81 (m, 2H), 1.21 (d, *J* = 6.87 Hz, 3H). MS (ESI): [M + H]⁺ = 507.3

### (12) Synthesis of (S)-3-(4-((1-cyclopentyl-3-(3,5-dichloro-4-hydroxyphenyl)-1H-indazol-6-yl)methoxy)phenyl)butanoic acid (Compound 44)

Compound 44 was synthesized from C3 and (3,5-dichloro-4-hydroxyphenyl)boronic acid in the same manner as described for the synthesis of Compound 10, to afford a white solid (38% yield).

¹H NMR (CDCl₃, 400 MHz): δ 7.93 (d, *J =* 8.55 Hz, 1H), 7.87 - 7.90 (m, 2H), 7.57 (s, 1H), 7.24 - 7.27 (m, 1H), 7.18 (d, *J =* 8.55 Hz, 2H), 6.93 - 7.02 (m, 2H), 5.21 (s, 2H), 4.97 - 5.09 (m, 1H), 3.20 - 3.32 (m, 1H), 2.54 - 2.70 (m, 2H), 2.16 - 2.27 (m, 4H), 1.99 - 2.05 (m, 2H), 1.74 - 1.81 (m, 2H), 1.32 (d, *J =* 7.02 Hz, 3H). MS (ESI): [M + H]⁺ = 539.2

### (13) Synthesis of (S)-3-(4-((1-cyclopentyl-3-(2,5-difluoro-4-hydroxyphenyl)-1H-indazol-6-yl)methoxy)phenyl)butanoic acid (Compound 45)

Compound 45 was synthesized from C3 and (2,5-difluoro-4-hydroxyphenyl)boronic acid in the same manner as described for the synthesis of Compound 10, to afford a white solid (35% yield).

¹H NMR (CDCl₃, 400 MHz): δ 7.80 (dd, *J* = 3.43, 8.47 Hz, 1H), 7.50 - 7.58 (m, 2H), 7.14 - 7.24 (m, 3H), 6.97 (d, *J* = 8.70 Hz, 2H), 6.89 (dd, *J* = 7.33, 10.53 Hz, 1H), 5.20 (s, 2H), 5.00 - 5.09 (m, 1H), 3.22 - 3.32 (m, 1H), 2.55 - 2.69 (m, 2H), 2.15 - 2.28 (m, 4H), 1.95 - 2.02 (m, 2H), 1.71 - 1.80 (m, 2H), 1.31 (d, *J =* 7.33 Hz, 3H). MS (ESI): [M + H]⁺ = 507.3

### (14) Synthesis of (S)-3-(4-((1-cyclopentyl-3-(2,5-dichloro-4-hydroxyphenyl)-1H-indazol-6-yl)methoxy)phenyl)butanoic acid (Compound 46)

Compound 46 was synthesized from C3 and (2,5-dichloro-4-hydroxyphenyl)boronic acid in the same manner as described for the synthesis of Compound 10, to afford a white solid (42% yield).

¹H NMR (CDCl₃, 400 MHz): δ 7.64 (d, *J =* 8.70 Hz, 1H), 7.52 - 7.57 (m, 2H), 7.12 - 7.22 (m, 4H), 6.90 - 7.00 (m, 2H), 5.18 (s, 2H), 5.02 (quin, *J* = 7.21 Hz, 1H), 3.19 - 3.28 (m, 1H), 2.59 (dq, *J* = 7.56, 15.80 Hz, 2H), 2.09 - 2.30 (m, 4H), 1.92 - 2.06 (m, 2H), 1.63 - 1.77 (m, 2H), 1.29 (d, *J* = 6.87 Hz, 3H). MS (ESI): [M + H]⁺ = 539.2

### (15) Synthesis of (S)-3-(4-((1-cyclopentyl-3-(3,5-difluoro-4-(hydroxymethyl)phenyl)-1H-indazol-6-yl)methoxy)phenyl)butanoic acid (Compound 47)

Compound 47 was synthesized from C3 and (3,5-difluoro-4-(hydroxymethyl)phenyl)boronic acid in the same manner as described for the synthesis of Compound 10, to afford a white solid (57% yield).

¹H NMR (METHANOL-d₄, 400 MHz) δ 8.03 (d, *J =* 8.47 Hz, 1H), 7.72 (s, 1H), 7.59 (d, *J* = 8.70 Hz, 2H), 7.35 (dd, *J* = 1.14, 8.47 Hz, 1H), 7.15 - 7.21 (m, 2H), 6.92 - 7.00 (m, 2H), 5.24 (s, 2H), 5.18 (t, *J* = 7.10 Hz, 1H), 4.73 (s, 2H), 3.13 - 3.23 (m, 1H), 2.52 (dd, *J =* 4.58, 7.56 Hz, 2H), 2.15 - 2.27 (m, 4H), 1.97 - 2.05 (m, 2H), 1.76 - 1.87 (m, 2H), 1.27 (d, *J* = 6.87 Hz, 3H). MS (ESI): [M + H]⁺ = 521.3

### (16) Synthesis of (S)-3-(4-((1-cyclopentyl-3-(3,5-dichloro-4-(hydroxymethyl)phenyl)-1H-indazol-6-yl)methoxy)phenyl)butanoic acid (Compound 48)

Compound 48 was synthesized from C3 and (3,5-dichloro-4-(hydroxymethyl)phenyl)boronic acid in the same manner as described for the synthesis of Compound 10, to afford a white solid (45% yield).

¹H NMR (METHANOL-d₄, 400 MHz) δ 7.97 - 8.02 (m, 3H), 7.74 (s, 1H), 7.34 - 7.38 (m, 1H), 7.14 - 7.22 (m, 2H), 6.92 - 7.01 (m, 2H), 5.24 (s, 2H), 5.13 - 5.22 (m, 1H), 4.91 - 4.98 (m, 2H), 3.13 - 3.24 (m, 1H), 2.47 - 2.55 (m, 2H), 2.16 - 2.28 (m, 4H), 2.00 - 2.06 (m, 2H), 1.77 - 1.86 (m, 2H), 1.23 - 1.29 (m, 3H). MS (ESI): [M + H]⁺ = 553.00

### (17) Synthesis of (S)-3-(4-((1-cyclopentyl-3-(4-methoxyphenyl)-1H-indazol-6-yl)methoxy)phenyl)butanoic acid (Compound 49)

Compound 49 was synthesized from C3 and (4-methoxyphenyl)boronic acid in the same manner as described for the synthesis of Compound 10, to afford a white solid (31% yield).

### (18) Synthesis of (S)-3-(4-((1-cyclopentyl-3-(4-nitrophenyl)-1H-indazol-6-yl)methoxy)phenyl)butanoic acid (Compound 50)

Compound 50 was synthesized from C3 and (4-nitrophenyl)boronic acid in the same manner as described for the synthesis of Compound 10, to afford a white solid (41% yield).

### (19) Synthesis of (S)-4-(6-((4-(1-carboxypropan-2-yl)phenoxy)methyl)-1-cyclopentyl-1H-indazol-3-yl)benzoic acid (Compound 51)

Compound 51 was synthesized from C3 and 4-boronobenzoic acid in the same manner as described for the synthesis of Compound 10, to afford a white solid (15% yield).

### (20) Synthesis of (S)-4-(6-((4-(1-carboxypropan-2-yl)phenoxy)methyl)-1-cyclopentyl-1H-indazol-3-yl)-3-fluorobenzoic acid (Compound 52)

Compound 52 was synthesized from C3 and 4-borono-3-fluorobenzoic acid in the same manner as described for the synthesis of Compound 10, to afford a white solid (11% yield).

### (21) Synthesis of (S)-3-(4-((1-cyclopentyl-3-(4-hydroxy-3,5-dimethylphenyl)-1H-indazol-6-yl)methoxy)phenyl)butanoic acid (Compound 53)

Compound 53 was synthesized from C3 and (4-hydroxy-3,5-dimethylphenyl)boronic acid in the same manner as described for the synthesis of Compound 10, to afford a white solid (35% yield).

### (22) Synthesis of (S)-3-(4-((1-cyclopentyl-3-(3-(hydroxymethyl)phenyl)-1H-indazol-6-yl)methoxy)phenyl)butanoic acid (Compound 54)

Compound 54 was synthesized from C3 and (3-(hydroxymethyl)phenyl)boronic acid in the same manner as described for the synthesis of Compound 10, to afford a white solid (47% yield).

### (23) Synthesis of (S)-3-(4-((1-cyclopentyl-3-(2,3-difluoro-4-hydroxyphenyl)-1H-indazol-6-yl)methoxy)phenyl)butanoic acid (Compound 55)

Compound 55 was synthesized from C3 and (2,3-difluoro-4-hydroxyphenyl)boronic acid in the same manner as described for the synthesis of Compound 10, to afford a white solid (35% yield).

### Preparative Example 4. Synthesis of Compounds 56 to 58

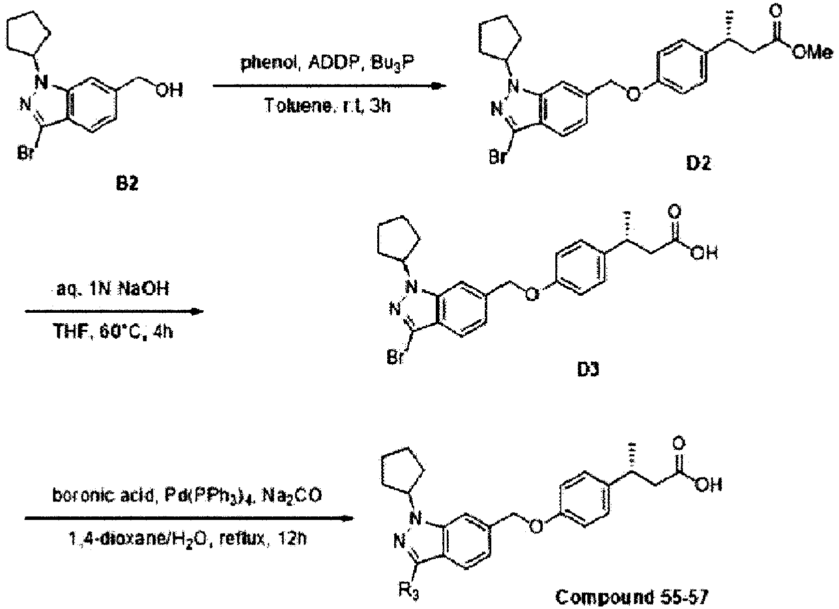

### (1) Synthesis of methyl (R)-3-(4-((3-bromo-1-cyclopentyl-1H-indazol-6-yl)methoxy)phenyl)butanoate (Compound D2)

D1 (0.034 mmol) was dissolved in toluene (2 mL), and (R)-3-(4-hydroxyphenyl)butanoate (0.051 mmol) was added. Subsequently, tributylphosphine (25 µL, 0.102 mmol) and 1,1'-(azodicarbonyl)dipiperidine (26 mg, 0.102 mmol) were added, and the mixture was stirred at room temperature for 3 h. The reaction mixture was then filtered and washed with hexane. The filtrate was then concentrated under reduced pressure to yield a crude product. The crude product was purified by silica gel column chromatography (hexane:ethyl acetate = 8:1) to obtain Compound D2 as a colorless oil (53% yield).

### (2) Synthesis of (R)-3-(4-((3-bromo-1-cyclopentyl-1H-indazol-6-yl)methoxy)phenyl)butanoic acid (Compound D3)

D2 was dissolved in THF (0.3 mL). 1 N aqueous NaOH solution (0.1 mL) was added dropwise, and the mixture was stirred at 60°C for 4 h. The reaction mixture was then poured into 1 N aqueous HCl solution and extracted with ethyl acetate. The combined extracts were dried over Na₂SO₄, filtered, and concentrated under reduced pressure to yield a crude product. The crude product was purified by silica gel column chromatography (CHCl₃:MeOH = 20:1) to obtain Compound D3 as a white solid (71% yield).

### (3) Synthesis of (R)-3-(4-((1-cyclopentyl-3-(4-hydroxyphenyl)-1H-indazol-6-yl)methoxy)phenyl)butanoic acid (Compound 56)

Compound 56 was synthesized from D3 and (4-hydroxyphenyl)boronic acid in the same manner as described for the synthesis of Compound 10, to afford a white solid (32% yield).

### (4) Synthesis of (R)-3-(4-((1-cyclopentyl-3-(4-methoxyphenyl)-1H-indazol-6-yl)methoxy)phenyl)butanoic acid (Compound 57)

Compound 57 was synthesized from D3 and (4-methoxyphenyl)boronic acid in the same manner as described for the synthesis of Compound 10, to afford a white solid (45% yield).

### (5) Synthesis of (R)-3-(4-((1-cyclopentyl-3-(4-nitrophenyl)-1H-indazol-6-yl)methoxy)phenyl)butanoic acid (Compound 58)

Compound 58 was synthesized from D3 and (4-nitrophenyl)boronic acid in the same manner as described for the synthesis of Compound 10, to afford a white solid (30% yield).

### Preparative Example 5. Synthesis of Compounds 59 to 62

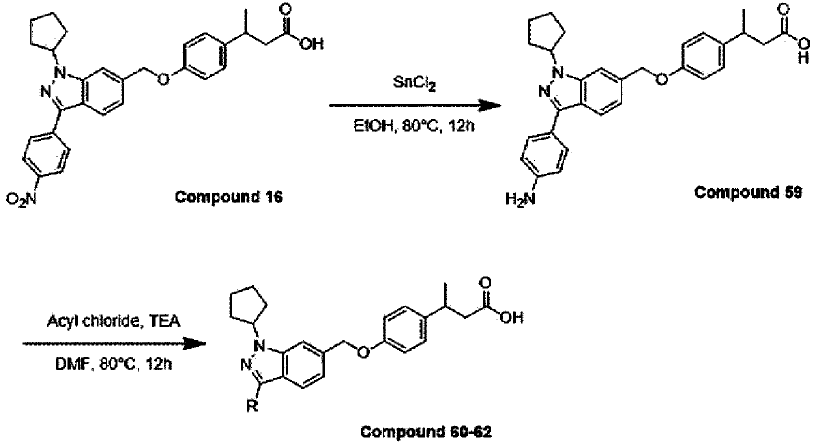

### (1) Synthesis of 3-(4-((3-(4-aminophenyl)-1-cyclopentyl-1H-indazol-6-yl)methoxy)phenyl)butanoic acid (Compound 59)

Compound 16 (50 mg, 0.1 mmol) and SnCl₂ (56 mg, 0.3 mmol) were dissolved in EtOH (2 mL). The reaction mixture was stirred at 80°C for 12 h. The reaction mixture was poured into water and extracted with ethyl acetate. The combined extracts were dried over Na₂SO₄, filtered, and concentrated under reduced pressure to yield a crude product. The crude product was purified by silica gel column chromatography (hexane:ethyl acetate = 4:1) to obtain Compound 59 as a white solid (23 mg, 50% yield).

### (2) Synthesis of 3-(4-((3-(4-acetamidophenyl)-1-cyclopentyl-1H-indazol-6-yl)methoxy)phenyl)butanoic acid (Compound 60)

Compound 59 (23 mg, 0.05 mmol), acetyl chloride (7.8 mg, 0.1 mmol), and trimethylamine (10 mg) were dissolved in DMF (2 mL). The reaction mixture was stirred at 80°C for 12 h. The reaction mixture was poured into water and extracted with ethyl acetate. The combined extracts were dried over Na₂SO₄, filtered, and concentrated under reduced pressure to yield a crude product. The crude product was purified by silica gel column chromatography (hexane:ethyl acetate = 1:1) to obtain Compound 60 as a white solid (3.8 mg, 15% yield).

### (3) Synthesis of 3-(4-((3-(4-acetamidophenyl)-1-cyclopentyl-1H-indazol-6-yl)methoxy)phenyl)butanoic acid (Compound 61)

Compound 61 was synthesized from Compound 59 and propionyl chloride in the same manner as described for the synthesis of Compound 60, to afford a white solid (12% yield).

### (4) Synthesis of 3-(4-((3-(4-(but-3-enamido)phenyl)-1-cyclopentyl-1H-indazol-6-yl)methoxy)phenyl)butanoic acid (Compound 62)

Compound 62 was synthesized from Compound 59 and but-3-enoyl chloride in the same manner as described for the synthesis of Compound 60, to afford a white solid (10% yield).

### Preparative Example 6. Synthesis of Compounds 63 to 67

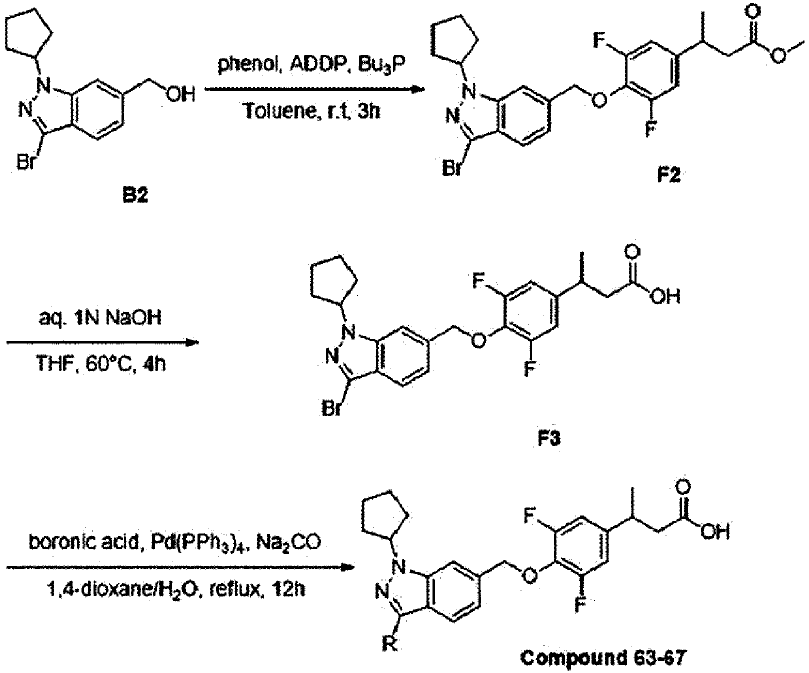

### (1) Synthesis of methyl 3-(4-((3-bromo-1-cyclopentyl-1H-indazol-6-yl)methoxy)-3,5-difluorophenyl)butanoate (F2)

F2 was synthesized from B2 and methyl 3-(3,5-difluoro-4-hydroxyphenyl)butanoate in the same manner as described in the synthesis of B3 in Preparative Example 2, to afford a white solid (35% yield).

### (2) Synthesis of 3-(4-((3-bromo-1-cyclopentyl-1H-indazol-6-yl)methoxy)-3,5-difluorophenyl)butanoic acid (F3)

F3 was synthesized from F2 in the same manner as described in the synthesis of B4 in Preparative Example 2, to afford a white solid (73% yield).

### (3) Synthesis of 3-(4-((1-cyclopentyl-3-(4-hydroxyphenyl)-1H-indazol-6-yl)methoxy)-3,5-difluorophenyl)butanoic acid (Compound 63)

Compound 63 was synthesized from F3 and (4-hydroxyphenyl)boronic acid in the same manner as described for the synthesis of Compound 10, to afford a white solid (47% yield).

### (4) Synthesis of 3-(4-((3-(3-chloro-4-hydroxyphenyl)-1-cyclopentyl-1H-indazol-6-yl)methoxy)-3,5-difluorophenyl)butanoic acid (Compound 64)

Compound 64 was synthesized from F3 and (3-chloro-4-hydroxyphenyl)boronic acid in the same manner as described for the synthesis of Compound 10, to afford a white solid (24% yield).

### (5) Synthesis of 3-(4-((1-cyclopentyl-3-(4-hydroxy-3-(trifluoromethyl)phenyl)-1H-indazol-6-yl)methoxy)-3,5-difluorophenyl)butanoic acid (Compound 65)

Compound 65 was synthesized from F3 and (4-hydroxy-3-(trifluoromethyl)phenyl)boronic acid in the same manner as described for the synthesis of Compound 10, to afford a white solid (35% yield).

### (6) Synthesis of 3-(4-((1-cyclopentyl-3-(3,5-difluoro-4-hydroxyphenyl)-1H-indazol-6-yl)methoxy)-3,5-difluorophenyl)butanoic acid (Compound 66)

Compound 66 was synthesized from F3 and (3,5-difluoro-4-hydroxyphenyl)boronic acid in the same manner as described for the synthesis of Compound 10, to afford a white solid (27% yield).

### (7) Synthesis of 3-(4-((1-cyclopentyl-3-(3,5-dichloro-4-hydroxyphenyl)-1H-indazol-6-yl)methoxy)-3,5-difluorophenyl)butanoic acid (Compound 67)

Compound 67 was synthesized from F3 and (3,5-dichloro-4-hydroxyphenyl)boronic acid in the same manner as described for the synthesis of Compound 10, to afford a white solid (30% yield).

### Preparative Example 7. Synthesis of Compounds 68 to 71

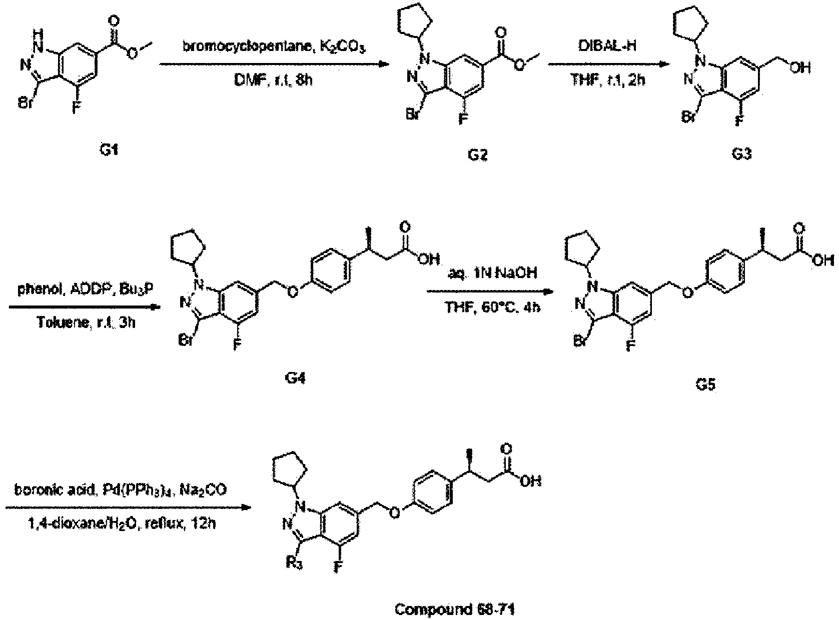

### (1) Synthesis of methyl 3-bromo-1-cyclopentyl-4-fluoro-1H-indazole-6-carboxylate (G2)

G2 was synthesized from methyl 3-bromo-4-fluoro-1H-indazole-6-carboxylate (G1) in the same manner as described in the synthesis of B1 in Preparative Example 2, to afford a colorless oil (43% yield).

¹H NMR (DMSO-d₆, 400 MHz) δ 8.29 (d, *J =* 0.92 Hz, 1H), 7.40 (dd, *J* = 0.92, 10.99 Hz, 1H), 5.32 - 5.41 (m, 1H), 3.92 (s, 3H), 2.06 - 2.24 (m, 2H), 1.90 - 2.02 (m, 2H), 1.79 - 1.90 (m, 2H), 1.60 - 1.76 (m, 2H). MS (ESI): [M + H]⁺ = 341.0

### (2) Synthesis of (3-bromo-1-cyclopentyl-4-fluoro-1H-indazol-6-yl)methanol (G3)

G3 was synthesized from G2 in the same manner as described in the synthesis of B2 in Preparative Example 2, to afford a colorless oil (35% yield).

¹H NMR (DMSO-d₆, 400 MHz) δ 8.53 (s, 1H), 7.30 - 7.36 (m, 1H), 6.71 (d, *J* = 11.60 Hz, 1H), 4.96 - 5.05 (m, 1H), 4.53 (s, 2H), 2.13 - 2.23 (m, 2H), 2.02 - 2.13 (m, 2H), 1.82 - 1.94 (m, 2H), 1.65 - 1.76 (m, 2H). MS (ESI): [M + H]⁺ = 313.0

### (3) Synthesis of methyl (S)-3-(4-((3-bromo-1-cyclopentyl-4-fluoro-1H-indazol-6-yl)methoxy)phenyl)butanoate (G4)

G4 was synthesized from G3 in the same manner as described in the synthesis of B3 in Preparative Example 2, to afford a yellow oil (65% yield).

¹H NMR (CDCl₃, 400 MHz): δ 7.30 (s, 1H), 7.12 - 7.19 (m, 2H), 6.90 - 6.98 (m, 2H), 6.85 (d, *J =* 10.53 Hz, 1H), 5.14 (s, 2H), 4.91 (quin, *J =* 7.38 Hz, 1H), 3.63 (s, 3H), 3.20 - 3.32 (m, 1H), 2.47 - 2.64 (m, 2H), 2.06 - 2.22 (m, 4H), 1.83 - 2.01 (m, 2H), 1.65 - 1.81 (m, 2H), 1.28 (d, *J =* 6.87 Hz, 3H). MS (ESI): [M + H]⁺ = 489.2

### (5) Synthesis of (S)-3-(4-((3-bromo-1-cyclopentyl-4-fluoro-1H-indazol-6-yl)methoxy)phenyl)butanoic acid (G5)

G5 was synthesized from G4 in the same manner as described in the synthesis of B4 in Preparative Example 2, to afford a colorless oil (40% yield).

¹H NMR (CDCl₃, 400 MHz): δ 7.30 (s, 1H), 7.13 - 7.20 (m, 2H), 6.90 - 6.97 (m, 2H), 6.85 (d, *J =* 10.76 Hz, 1H), 5.14 (s, 2H), 4.91 (quin, *J* = 7.33 Hz, 1H), 3.12 - 3.31 (m, 1H), 2.51 - 2.67 (m, 2H), 2.08 - 2.23 (m, 4H), 1.90 - 2.06 (m, 2H), 1.68 - 1.81 (m, 2H), 1.31 (d, *J =* 6.87 Hz, 3H). MS (ESI): [M + H]⁺ = 476.2

### (6) Synthesis of (S)-3-(4-((1-cyclopentyl-3-(3,5-difluoro-4-hydroxyphenyl)-4-fluoro-1H-indazol-6-yl)methoxy)phenyl)butanoic acid (Compound 68)

Compound 68 was synthesized from G5 and (3,5-difluoro-4-hydroxyphenyl)boronic acid in the same manner as described for the synthesis of Compound 10, to afford a white solid (41% yield).

¹H NMR (METHANOL-d₄, 400 MHz) δ ppm 7.45 - 7.52 (m, 3 H), 7.15 - 7.20 (m, 2 H), 6.92 - 7.00 (m, 3 H), 5.15 - 5.24 (m, 2 H), 5.06 - 5.14 (m, 1 H), 3.11 - 3.24 (m, 1 H), 2.46 - 2.56 (m, 2 H), 2.13 - 2.27 (m, 4 H), 1.96 - 2.04 (m, 2 H), 1.76 - 1.86 (m, 2 H), 1.25 - 1.28 (m, 3 H). MS (ESI): [M + H]⁺ = 525.3

### (7) Synthesis of (S)-3-(4-((1-cyclopentyl-3-(3,5-dichloro-4-hydroxyphenyl)-4-fluoro-1H-indazol-6-yl)methoxy)phenyl)butanoic acid (Compound 69)

Compound 69 was synthesized from G5 and (3,5-dichloro-4-hydroxyphenyl)boronic acid in the same manner as described for the synthesis of Compound 10, to afford a white solid (45% yield).

¹H NMR (METHANOL-d₄, 400 MHz) δ ppm 7.78 - 7.82 (m, 2 H), 7.48 (s, 1 H), 7.13 - 7.21 (m, 2 H), 6.91 - 6.99 (m, 3 H), 5.17 (s, 2 H), 5.11 (quin, *J*=7.10 Hz, 1 H), 3.10 - 3.21 (m, 1 H), 2.49 - 2.56 (m, 2 H), 2.09 - 2.23 (m, 4 H), 1.88 - 2.06 (m, 2 H), 1.75 - 1.84 (m, 2 H), 1.23 - 1.29 (m, 3 H). MS (ESI): [M + H]⁺ = 557.1

### (8) Synthesis of (S)-3-(4-((1-cyclopentyl-3-(3,5-difluoro-4-(hydroxymethyl)phenyl)-4-fluoro-1H-indazol-6-yl)methoxy)phenyl)butanoic acid (Compound 70)

Compound 70 was synthesized from G5 and (3,5-difluoro-4-(hydroxymethyl)phenyl)boronic acid in the same manner as described for the synthesis of Compound 10, to afford a white solid (58% yield).

¹H NMR (METHANOL-*d*₄, 400 MHz) δ ppm 7.49 - 7.59 (m, 3 H), 7.14 - 7.21 (m, 2 H), 6.93 - 7.02 (m, 3 H), 5.19 (s, 2 H), 5.10 - 5.18 (m, 1 H), 4.72 (s, 2 H), 3.11 - 3.23 (m, 1 H), 2.47 - 2.59 (m, 2 H), 2.10 - 2.27 (m, 4 H), 1.96 - 2.05 (m, 2 H), 1.75 - 1.88 (m, 2 H), 1.26 (d, *J*=6.87 Hz, 3 H). MS (ESI): [M + H]⁺ = 539.3

### (9) Synthesis of (S)-3-(4-((1-cyclopentyl-3-(3,5-dichloro-4-(hydroxymethyl)phenyl)-4-fluoro-1H-indazol-6-yl)methoxy)phenyl)butanoic acid (Compound 71)

Compound 71 was synthesized from G5 and (3,5-dichloro-4-(hydroxymethyl)phenyl)boronic acid in the same manner as described for the synthesis of Compound 10, to afford a white solid (36% yield).

¹H NMR (METHANOL-*d*₄, 400 MHz) δ ppm 7.92 (s, 2 H), 7.52 (s, 1 H), 7.13 - 7.21 (m, 2 H), 6.92 - 7.01 (m, 3 H), 5.17 (s, 2 H), 5.10 - 5.16 (m, 1 H), 4.91 (s, 2 H), 3.11 - 3.22 (m, 1 H), 2.43 - 2.57 (m, 2 H), 2.10 - 2.25 (m, 4 H), 1.90 - 2.06 (m, 2 H), 1.71 - 1.86 (m, 2 H), 1.26 (d, *J*=6.87 Hz, 3 H). MS (ESI): [M + H]⁺ = 571.2

### Preparative Example 8. Synthesis of Compounds 72 to 75

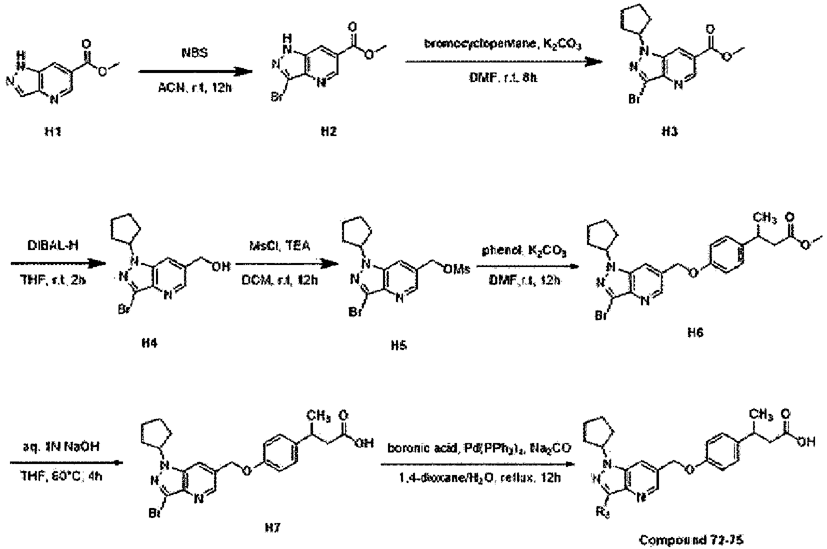

### (1) Synthesis of methyl 3-bromo-1H-pyrazolo[4,3-b]pyridine-6-carboxylate (H2)

Methyl 1H-pyrazolo[4,3-b]pyridine-6-carboxylate (H1) (20 mg, 0.113 mmol) and n-bromosuccinimide (22 mg, 0.124 mmol) were dissolved in ACN (2 mL). The reaction mixture was stirred at room temperature for 12 h. The reaction mixture was poured into water and extracted with ethyl acetate. The combined extracts were dried over Na₂SO₄, filtered, and concentrated under reduced pressure to yield a crude product. The crude product was purified by silica gel column chromatography (hexane:ethyl acetate = 1:1) to obtain Compound H2 as a colorless oil (8 mg, 30% yield).

### (2) Synthesis of methyl 3-bromo-1-cyclopentyl-1H-pyrazolo[4,3-b]pyridine-6-carboxylate (H3)

Compound H3 was synthesized from H2 in the same manner as described in the synthesis of B1 in Preparative Example 2, to afford a white solid (41% yield).

### (3) Synthesis of (3-bromo-1-cyclopentyl-1H-pyrazolo[4,3-b]pyridin-6-yl)methanol (H4)

Compound H4 was synthesized from H3 in the same manner as described in the synthesis of B2 in Preparative Example 2, to afford a white solid (36% yield).

### (4) Synthesis of (3-bromo-1-cyclopentyl-1H-pyrazolo[4,3-b]pyridin-6-yl)methyl methanesulfonate (H5)

Compound H4 (20 mg, 0.08 mmol), methanesulfonyl chloride (9 mg, 0.169 mmol), and trimethylamine (17 mg, 0.169 mmol) were dissolved in DCM (2 mL). The reaction mixture was stirred at room temperature for 12 h. The reaction mixture was poured into water and extracted with ethyl acetate. The combined extracts were dried over Na₂SO₄, filtered, and concentrated under reduced pressure to yield a crude product. The crude product was purified by silica gel column chromatography (hexane:ethyl acetate = 3:1) to obtain Compound H5 as a colorless oil (21 mg, 66% yield).

### (5) Synthesis of methyl 3-(4-((3-bromo-1-cyclopentyl-1H-pyrazolo[4,3-b]pyridin-6-yl)methoxy)phenyl)butanoate (H6)

Compound H5 (273 mg, 0.731 mmol), potassium carbonate (202 mg, 1.462 mmol), and methyl 3-(4-hydroxyphenyl)butanoate (160 mg, 0.804 mmol) were dissolved in DMF (2 mL). The reaction mixture was stirred at room temperature for 12 h. The reaction mixture was poured into water and extracted with ethyl acetate. The combined extracts were dried over Na₂SO₄, filtered, and concentrated under reduced pressure to yield a crude product. The crude product was purified by silica gel column chromatography (hexane:ethyl acetate = 5:1) to obtain Compound H6 as a colorless oil (237 mg, 68% yield).

### (6) Synthesis of 3-(4-((3-bromo-1-cyclopentyl-1H-pyrazolo[4,3-b]pyridin-6-yl)methoxy)phenyl)butanoic acid (H7)

Compound H7 was synthesized from H6 in the same manner as described in the synthesis of B3 in Preparative Example 2, to afford a white solid (56% yield).

### (7) Synthesis of 3-(4-((1-cyclopentyl-3-(4-hydroxyphenyl)-1H-pyrazolo[4,3-b]pyridin-6-yl)methoxy)phenyl)butanoic acid (Compound 72)

Compound 72 was synthesized from H7 and (4-hydroxyphenyl)boronic acid in the same manner as described for the synthesis of Compound 10, to afford a white solid (35% yield).

### (8) Synthesis of 3-(4-((1-cyclopentyl-3-(3,5-dichloro-4-hydroxyphenyl)-1H-pyrazolo[4,3-b]pyridin-6-yl)methoxy)phenyl)butanoic acid (Compound 73)

Compound 73 was synthesized from H7 and (3,5-dichloro-4-hydroxyphenyl)boronic acid in the same manner as described for the synthesis of Compound 10, to afford a white solid (41% yield).

### (9) Synthesis of 3-(4-((1-cyclopentyl-3-(3,5-difluoro-4-hydroxyphenyl)-1H-pyrazolo[4,3-b]pyridin-6-yl)methoxy)phenyl)butanoic acid (Compound 74)

Compound 74 was synthesized from H7 and (3,5-difluoro-4-hydroxyphenyl)boronic acid in the same manner as described for the synthesis of Compound 10, to afford a white solid (31% yield).

### (10) Synthesis of 3-(4-((3-(3-chloro-4-hydroxyphenyl)-1-cyclopentyl-1H-pyrazolo[4,3-b]pyridin-6-yl)methoxy)phenyl)butanoic acid (Compound 75)

Compound 75 was synthesized from H7 and (3-chloro-4-hydroxyphenyl)boronic acid in the same manner as described for the synthesis of Compound 10, to afford a white solid (43% yield).

### Preparative Example 9. Synthesis of Compounds 76 to 86

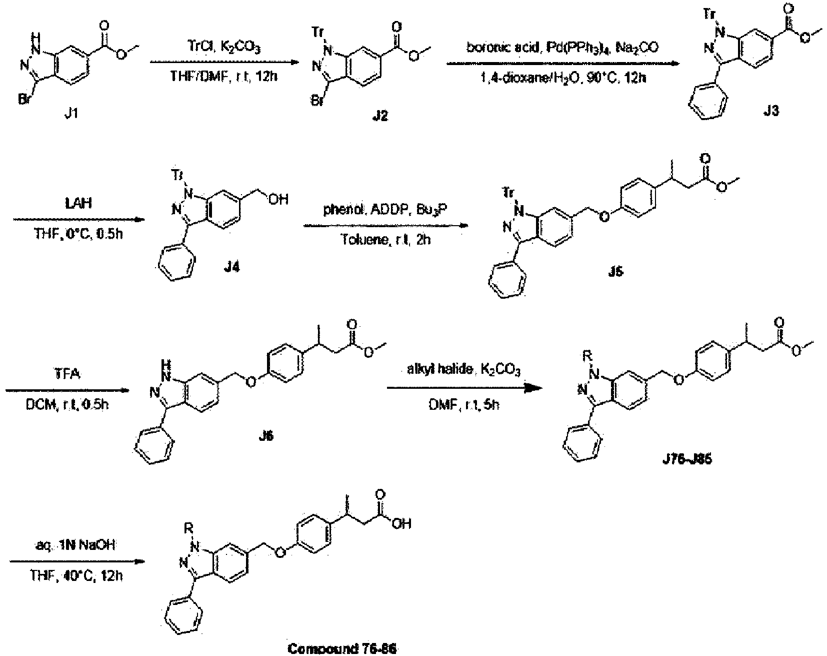

### (1) Synthesis of methyl 3-bromo-1-trityl-1H-indazole-6-carboxylate (Compound J2)

Methyl 3-bromo-1H-indazole-6-carboxylate (J1) (20 mg, 0.078 mmol), trityl chloride (23 mg, 0.082 mmol), and K₂CO₃ (32 mg, 0.235 mmol) were dissolved in THF (0.3 mL) and DMF (0.1 mL). The mixture was stirred at room temperature for 12 h. The reaction mixture was evaporated under reduced pressure. The residue was dissolved in water and extracted with ethyl acetate. The combined extracts were dried over Na₂SO₄, filtered, and concentrated under reduced pressure to yield a crude product. The crude product was purified by silica gel column chromatography (CHCl₃:hexane:DCM = 30:1:1) to obtain Compound J2 as a white solid (22 mg, 57% yield).

¹H NMR (DMSO-d₆, 400 MHz) δ 7.72 (d, *J* = 0.92 Hz, 2H), 7.30 - 7.39 (m, 9H), 7.13 - 7.17 (m, 6H), 7.03 (t, *J =* 1.07 Hz, 1H), 3.72 (s, 3H)

### (2) Synthesis of methyl 3-phenyl-1-trityl-1H-indazole-6-carboxylate (Compound J3)

Compound J2 (1.5 g, 3.016 mmol), phenylboronic acid (411 mg, 3.62 mmol), Pd(PPh₃)₄ (174 mg, 0.15 mmol), and Na₂CO₃ (959 mg, 9.047 mmol) were added to a solution of 1,4-dioxane (25 mL) and water (5 mL), and the mixture was stirred under reflux for 4 h. The reaction mixture was evaporated under reduced pressure. The residue was dissolved in water and extracted with ethyl acetate. The combined extracts were dried over Na₂SO₄, filtered, and concentrated under reduced pressure to yield a crude product. The crude product was purified by silica gel column chromatography (CHCl₃-:hexane = 2:1) to obtain Compound J3 as a white solid (1.25 g, 84% yield).

¹H NMR (DMSO-*d*₆, 400 MHz) δ ppm 8.20 (dd, *J*=8.55, 0.61 Hz, 1 H), 7.85 - 7.92 (m, 2 H), 7.67 - 7.77 (m, 1 H), 7.49 - 7.55 (m, 2 H), 7.40 - 7.46 (m, 1 H), 7.30 - 7.39 (m, 9 H), 7.19 - 7.25 (m, 6 H), 7.10 - 7.13 (m, 1 H), 3.73 (s, 3 H)

### (3) Synthesis of (3-phenyl-1-trityl-1H-indazol-6-yl)methanol (Compound J4)

Compound J3 (50 mg, 0.101 mmol) was dissolved in THF (1 mL) and cooled to 0°C. A 1.0 M THF solution of lithium aluminum hydride (202 µL, 0.202 mmol) was added dropwise to the solution. The reaction mixture was stirred at 0°C for 30 min. The reaction mixture was poured into saturated aqueous NH₄Cl solution and then extracted with ethyl acetate. The combined extracts were dried over Na₂SO₄, filtered, and concentrated under reduced pressure to yield a crude product. The crude product was purified by silica gel column chromatography (CHCl₃:hexane = 40:1) to obtain Compound J4 as a white solid (38 mg, 81% yield).

¹H NMR (DMSO-*d*₆, 400 MHz) δ ppm 8.01 (d, *J*=8.55 Hz, 1 H), 7.85 - 7.89 (m, 2 H), 7.46 - 7.51 (m, 2 H), 7.28 - 7.42 (m, 10 H), 7.19 - 7.27 (m, 6 H), 7.11 (d, *J*=8.55 Hz, 1 H), 6.45 - 6.48 (m, 1 H), 5.07 - 5.13 (m, 1 H), 4.32 (d, *J*=4.27 Hz, 2 H)

### (4) Synthesis of methyl 3-(4-((3-phenyl-1-trityl-1H-indazol-6-yl)methoxy)phenyl)butanoate (Compound J5)

Compound J4 (8 mg, 0.017 mmol) and methyl 3-(4-hydroxyphenyl)butanoate (5 mg, 0.026 mmol) were dissolved in toluene (1 mL). 1,1'-(azodicarbonyl)dipiperidine (13 mg, 0.051 mmol) and tributylphosphine (13 µL, 0.051 mmol) were added to the reaction mixture, and the mixture was stirred at room temperature for 2 h. The reaction mixture was filtered and washed with hexane. The filtrate was concentrated under reduced pressure to yield a crude product. The crude product was purified by silica gel column chromatography (hexane:ethyl acetate = 4:1) to obtain Compound J5 as a white solid. (10 mg, 91% yield).

¹H NMR (CDCl₃, 400 MHz) δ ppm 8.00 - 8.05 (m, 1 H), 7.90 - 7.94 (m, 2 H), 7.42 - 7.47 (m, 2 H), 7.32 - 7.38 (m, 1 H), 7.26 (s, 15 H), 7.19 (dd, *J*=8.39, 1.37 Hz, 1 H), 7.05 - 7.11 (m, 2 H), 6.68 - 6.75 (m, 2 H), 6.46 (s, 1 H), 4.86 (s, 2 H), 3.63 (s, 3 H), 3.19 - 3.27 (m, 1 H), 2.51 - 2.61 (m, 2 H), 1.27 (d, *J*=7.02 Hz, 3 H)

### (5) Synthesis of methyl 3-(4-((3-phenyl-1H-indazol-6-yl)methoxy)phenyl)butanoate (Compound J6)

Compound J5 (10 mg, 0.016 mmol) was dissolved in DCM (2 mL). TFA (20 µL) was added dropwise at 0°C, and the reaction mixture was stirred at room temperature for 30 min. The reaction mixture was concentrated under reduced pressure to yield a crude product. The crude product was purified by silica gel column chromatography (CHCl₃:MeOH = 50:1) to obtain Compound J6 as a white solid (3.7 mg, 58% yield).

¹H NMR (CDCl₃, 400 MHz) δ ppm 8.02 - 8.07 (m, 1 H), 7.93 - 8.01 (m, 2 H), 7.58 - 7.62 (m, 1 H), 7.50 - 7.55 (m, 2 H), 7.40 - 7.46 (m, 1 H), 7.28 - 7.31 (m, 1 H), 7.13 - 7.19 (m, 2 H), 6.91 - 6.97 (m, 2 H), 5.20 - 5.24 (m, 2 H), 3.60 - 3.65 (m, 3 H), 3.21 - 3.30 (m, 1 H), 2.50 - 2.62 (m, 2 H), 1.26 - 1.32 (m, 3 H). MS (ESI): [M + H]⁺ = 400.95

### (6) Synthesis of methyl 3-(4-((1-methyl-3-phenyl-1H-indazol-6-yl)methoxy)phenyl)butanoate (Compound J76)

Compound J6 (20 mg, 0.050 mmol) and K₂CO₃ (21 mg, 0.150 mmol) were dissolved in DMF (0.25 mL). Iodomethane (8 µL, 0.125 mmol) was added dropwise to the solution. The reaction mixture was stirred at room temperature for 5 h, poured into water, and extracted with ethyl acetate. The combined extracts were dried over Na₂SO₄, filtered, and concentrated under reduced pressure to yield a crude product. The crude product was purified by silica gel column chromatography (hexane:ethyl acetate = 3:1) to obtain Compound J76, to afford a colorless oil (16.5 mg, 80% yield).

¹H NMR (CDCl₃, 400 MHz): δ 8.02 (d, *J* = 8.47 Hz, 1H), 7.94 - 7.99 (m, 2H), 7.48 - 7.54 (m, 3H), 7.38 - 7.44 (m, 1H), 7.24 - 7.27 (m, 1H), 7.14 - 7.20 (m, *J=* 8.70 Hz, 2H), 6.93 - 6.99 (m, *J =* 8.70 Hz, 2H), 5.22 (s, 2H), 4.14 (s, 3H), 3.63 (s, 3H), 3.15 - 3.32 (m, 1H), 2.57 (s, 2H), 1.29 (d, *J =* 7.10 Hz, 3H). MS (ESI): [M + H]⁺ = 415.10

### (7) Synthesis of 3-(4-((3-phenyl-1H-indazol-6-yl)methoxy)phenyl)butanoic acid (Compound 76)

Compound J6 (30 mg, 0.075 mmol) was dissolved in THF (0.3 mL). 1 N aqueous NaOH solution (0.100 mL) was added dropwise, and the mixture was stirred at 40°C for 12 h. The reaction mixture was poured into 1 N aqueous HCl solution and extracted with ethyl acetate. The combined extracts were dried over Na₂SO₄, filtered, and concentrated under reduced pressure to yield a crude product. The crude product was purified by silica gel column chromatography (CHCl₃:MeOH = 80:1) to obtain Compound 76 as a white solid (16.7 mg, 57% yield).

¹H NMR (METHANOL-d₄, 400 MHz): δ 8.02 (dd, *J* = 0.61, 8.55 Hz, 1H), 7.92 - 7.96 (m, 2H), 7.62 - 7.65 (m, 1H), 7.49 - 7.55 (m, 2H), 7.38 - 7.45 (m, 1H), 7.30 (dd, *J* = 1.53, 8.55 Hz, 1H), 7.15 - 7.20 (m, 2H), 6.93 - 6.99 (m, 2H), 5.23 (s, 2H), 3.12 - 3.22 (m, 1H), 2.47 - 2.58 (m, 2H), 1.27 (d, *J =* 7.00 Hz, 3H). MS (ESI): [M + H]⁺ = 386.98

### (8) Synthesis of methyl 3-(4-((1-ethyl-3-phenyl-1H-indazol-6-yl)methoxy)phenyl)butanoate (Compound J77)

Compound J77 was synthesized from J76 and iodoethane in the same manner as described for the synthesis of Compound J76, to afford a white solid (73% yield).

¹H NMR (CDCl₃, 400 MHz): δ 8.02 (dd, *J =* 0.76, 8.39 Hz, 1H), 7.94 - 7.98 (m, 2H), 7.48 - 7.55 (m, 3H), 7.38 - 7.43 (m, 1H), 7.25 (dd, *J =* 1.22, 8.24 Hz, 1H), 7.14 - 7.19 (m, 2H), 6.94 - 7.02 (m, 2H), 5.22 (s, 2H), 4.50 (q, *J* = 7.32 Hz, 2H), 3.60 - 3.65 (m, 3H), 3.18 - 3.32 (m, 1H), 2.49 - 2.64 (m, 2H), 1.53 - 1.57 (m, 3H), 1.29 (d, *J =* 7.02 Hz, 3H). MS (ESI): [M + H]⁺ = 429.01

### (9) Synthesis of 3-(4-((1-methyl-3-phenyl-1H-indazol-6-yl)methoxy)phenyl)butanoic acid (Compound 77)

Compound 77 was synthesized from Compound J76 in the same manner as described for the synthesis of Compound 76, to afford a white solid (62% yield).

¹H NMR (CDCl₃, 400 MHz): δ 8.02 (d, *J* = 8.47 Hz, 1H), 7.96 (dd, *J* = 0.92, 8.24 Hz, 2H), 7.47 - 7.57 (m, 3H), 7.37 - 7.45 (m, 1H), 7.25 (d, *J =* 1.14 Hz, 1H), 7.15 - 7.21 (m, 2H), 6.95 - 7.02 (m, 2H), 5.22 (s, 2H), 4.08 - 4.16 (m, 3H), 3.19 - 3.31 (m, 1H), 2.46 - 2.73 (m, 2H), 1.32 (d, *J =* 6.87 Hz, 3H). MS (ESI): [M + H]⁺ = 401.01

### (10) Synthesis of methyl 3-(4-((3-phenyl-1-propyl-1H-indazol-6-yl)methoxy)phenyl)butanoate (Compound J78)

Compound J78 was synthesized from J6 and 1-iodopropane in the same manner as described for the synthesis of Compound J76, to afford a colorless oil (90% yield).

¹H NMR (CDCl₃, 400 MHz): δ 8.02 (d, *J* = 8.47 Hz, 1H), 7.94 - 7.98 (m, 2H), 7.48 - 7.55 (m, 3H), 7.37 - 7.42 (m, 1H), 7.25 (dd, *J =* 1.03, 8.36 Hz, 1H), 7.14 - 7.19 (m, 2H), 6.95 - 7.01 (m, 2H), 5.22 (s, 2H), 4.40 (t, *J =* 7.10 Hz, 2H), 3.60 - 3.65 (m, 3H), 3.20 - 3.31 (m, 1H), 2.50 - 2.66 (m, 2H), 1.93 - 2.07 (m, 2H), 1.29 (d, *J =* 6.87 Hz, 3H), 0.97 (t, *J* = 7.44 Hz, 3H). MS (ESI): [M + H]⁺ = 443.10

### (11) Synthesis of 3-(4-((1-ethyl-3-phenyl-1H-indazol-6-yl)methoxy)phenyl)butanoic acid (Compound 78)

Compound 78 was synthesized from Compound J77 in the same manner as described for the synthesis of Compound 76, to afford a white solid (62% yield).

¹H NMR (CDCl₃, 400 MHz): δ 8.00 - 8.04 (m, 1H), 7.93 - 7.99 (m, 2H), 7.48 - 7.54 (m, 3H), 7.37 - 7.43 (m, 1H), 7.25 (dd, *J =* 1.22, 8.55 Hz, 1H), 7.16 - 7.20 (m, 2H), 6.95 - 7.01 (m, 2H), 5.22 (s, 2H), 4.50 (q, *J* = 7.22 Hz, 2H), 3.23 - 3.32 (m, 1H), 2.61 (dq, *J* = 7.48, 15.51 Hz, 2H), 1.56 (t, *J* = 7.17 Hz, 3H), 1.32 (d, *J* = 7.02 Hz, 3H). MS (ESI): [M + H]⁺ = 415.04

### (12) Synthesis of methyl 3-(4-((1-isobutyl-3-phenyl-1H-indazol-6-yl)methoxy)phenyl)butanoate (Compound J79)

Compound J79 was synthesized from J6 and 1-iodo-2-methylpropanolin the same manner as described for the synthesis of Compound J76, to afford a colorless oil (71% yield).

¹H NMR (CDCl₃, 400 MHz): δ 8.01 (d, *J =* 8.24 Hz, 1H), 7.93 - 7.98 (m, 2H), 7.47 - 7.53 (m, 3H), 7.38 - 7.44 (m, 1H), 7.23 - 7.26 (m, 1H), 7.14 - 7.19 (m, 2H), 6.92 - 6.99 (m, 2H), 5.21 (s, 2H), 4.23 (d, *J =* 7.32 Hz, 2H), 3.63 (s, 3H), 3.22 - 3.32 (m, 1H), 2.51 - 2.65 (m, 2H), 2.37 - 2.43 (m, 1H), 1.28 (d, *J =* 6.71 Hz, 3H), 0.98 (d, *J =* 6.71 Hz, 6H). MS (ESI): [M + H]⁺ = 457.19

### (13) Synthesis of 3-(4-((3-phenyl-1-propyl-1H-indazol-6-yl)methoxy)phenyl)butanoic acid (Compound 79)

Compound 79 was synthesized from Compound J78 in the same manner as described for the synthesis of Compound 76, to afford a white solid (34% yield).

¹H NMR (CDCl₃, 400 MHz): δ 8.01 (dd, *J* = 0.46, 8.24 Hz, 1H), 7.93 - 7.97 (m, 2H), 7.47 - 7.54 (m, 3H), 7.36 - 7.43 (m, 1H), 7.25 (dd, *J =* 1.26, 8.36 Hz, 1H), 7.15 - 7.20 (m, 2H), 6.95 - 7.00 (m, 2H), 5.22 (s, 2H), 4.36 - 4.47 (m, 2H), 3.17 - 3.31 (m, 1H), 2.51-2.69 (m, 2H), 1.95 - 2.05 (m, 2H), 1.31 (d, *J =* 7.10 Hz, 3H), 0.92 - 1.00 (m, 3H). MS (ESI): [M + H]⁺ = 429.07

### (14) Synthesis of methyl 3-(4-((1-(cyclopropylmethyl)-3-phenyl-1H-indazol-6-yl)methoxy)phenyl)butanoate (Compound J80)

Compound J80 was synthesized from J6 and (bromomethyl)cyclopropane in the same manner as described for the synthesis of Compound J76, to afford a colorless oil (65% yield).

¹H NMR (CDCl₃, 400 MHz): δ 8.02 (dd, *J* = 0.69, 8.24 Hz, 1H), 7.94 - 7.99 (m, 2H), 7.47 - 7.55 (m, 3H), 7.36 - 7.43 (m, 1H), 7.25 (dd, *J =* 1.37, 8.24 Hz, 1H), 7.13 - 7.19 (m, 2H), 6.92 - 7.00 (m, 2H), 5.24 (s, 2H), 4.34 (d, *J =* 6.64 Hz, 2H), 3.63 (s, 3H), 3.18 - 3.30 (m, 1H), 2.46 - 2.64 (m, 2H), 1.35 - 1.45 (m, 1H), 1.28 (d, *J =* 7.10 Hz, 3H), 0.56 - 0.65 (m, 2H), 0.36 - 0.49 (m, 2H). MS (ESI): [M + H]⁺ = 455.12

### (15) Synthesis of 3-(4-((1-isobutyl-3-phenyl-1H-indazol-6-yl)methoxy)phenyl)butanoic acid (Compound 80)

Compound 80 was synthesized from Compound J79 in the same manner as described for the synthesis of Compound 76, to afford a white solid (93% yield).

¹H NMR (CDCl₃, 400 MHz): δ 8.01 (d, *J* = 8.55 Hz, 1H), 7.92 - 7.99 (m, 2H), 7.47 - 7.54 (m, 3H), 7.36 - 7.43 (m, 1H), 7.22 - 7.26 (m, 1H), 7.14 - 7.20 (m, 2H), 6.92 - 7.01 (m, 2H), 5.21 (s, 2H), 4.22 (d, *J =* 7.32 Hz, 2H), 3.12 - 3.30 (m, 1H), 2.50 - 2.71 (m, 2H), 2.32 - 2.46 (m, 1H), 1.31 (d, *J* = 6.60 Hz, 3H), 0.96 (d, *J* = 6.70 Hz, 6H)). MS (ESI): [M + H]⁺ = 443.10

### (16) Synthesis of methyl 3-(4-((1-cyclopentyl-3-phenyl-1H-indazol-6-yl)methoxy)phenyl)butanoate (Compound J81)

Compound J81 was synthesized from J6 and bromocyclopentane in the same manner as described for the synthesis of Compound J76, to afford a colorless oil (69% yield).

¹H NMR (CDCl₃, 400 MHz): δ 8.00 - 8.03 (m, 1H), 7.95 - 7.99 (m, 2H), 7.56 (s,1H), 7.47 - 7.52 (m, 2H), 7.35 - 7.41 (m, 1H), 7.24 (dd, *J =* 1.37, 8.47 Hz, 1H), 7.14 - 7.20 (m, 2H), 6.92 - 7.01 (m, 2H), 5.21 (s, 2H), 4.98 - 5.10 (m, 1H), 3.64 (s, 3H), 3.20 - 3.31 (m, 1H), 2.50 - 2.64 (m, 2H), 2.15 - 2.32 (m, 4H), 1.99 - 2.07 (m, 2H), 1.74 - 1.81 (m, 2H), 1.28 (d, *J =* 7.10 Hz, 3H). MS (ESI): [M + H]⁺ = 469.14

### (17) Synthesis of 3-(4-((1-(cyclopropylmethyl)-3-phenyl-1H-indazol-6-yl)methoxy)phenyl)butanoic acid (Compound 81)

Compound 81 was synthesized from Compound J80 in the same manner as described for the synthesis of Compound 76, to afford a white solid (97% yield).

¹H NMR (CDCl₃, 400 MHz): δ 8.02 (dd, *J* = 0.69, 8.47 Hz, 1H), 7.92 - 7.99 (m, 2H), 7.48 - 7.56 (m, 3H), 7.37 - 7.44 (m, 1H), 7.24 (dd, *J* = 1.14, 8.47 Hz, 1H), 7.15 - 7.22 (m, 2H), 6.93 - 7.02 (m, 2H), 5.23 (s, 2H), 4.23 - 4.36 (m, 2H), 3.20 - 3.31 (m, 1H), 2.61 (dq, *J =* 7.56, 15.42 Hz, 2H), 1.40 (s, 1H), 1.31 (d, *J* = 6.87 Hz, 3H), 0.53 - 0.62 (m, 2H), 0.38 - 0.49 (m, 2H). MS (ESI): [M + H]⁺ = 441.14

### (18) Synthesis of methyl 3-(4-((1-(cyclohexylmethyl)-3-phenyl-1H-indazol-6-yl)methoxy)phenyl)butanoate (Compound J82)

Compound J82 was synthesized from J6 and (bromomethyl)cyclohexane in the same manner as described for the synthesis of Compound J76, to afford a colorless oil (78% yield).

¹H NMR (CDCl₃, 400 MHz): δ 7.99 - 8.03 (m, 1H), 7.95 - 7.98 (m, 2H), 7.48 - 7.54 (m, 3H), 7.38 - 7.43 (m, 1H), 7.23 - 7.26 (m, 1H), 7.14 - 7.20 (m, 2H), 6.94 - 7.01 (m, 2H), 5.24 (s, 2H), 4.22 - 4.31 (m, 2H), 3.65 (s, 3H), 3.20 - 3.31 (m, 1H), 2.44 - 2.66 (m, 2H), 2.01 - 2.13 (m, 1H), 1.62 - 1.74 (m, 5H), 1.28 (d, *J =* 7.02 Hz, 3H), 0.99 - 1.24 (m, 5H). MS (ESI): [M + H]⁺ = 497.22

### (19) Synthesis of 3-(4-((1-cyclopentyl-3-phenyl-1H-indazol-6-yl)methoxy)phenyl)butanoic acid (Compound 82)

Compound 82 was synthesized from Compound J81 in the same manner as described for the synthesis of Compound 76, to afford a white solid (86% yield).

¹H NMR (CDCl₃, 400 MHz): δ 8.01 (d, *J* = 8.47 Hz, 1H), 7.95 - 7.99 (m, 2H), 7.56 (s, 1H), 7.47 - 7.52 (m, 2H), 7.35 - 7.41 (m, 1H), 7.24 (dd, *J =* 1.14, 8.47 Hz, 1H), 7.15 - 7.21 (m, 2H), 6.95 - 7.01 (m, 2H), 5.21 (s, 2H), 4.99 - 5.11 (m, 1H), 3.17 - 3.33 (m, 1H), 2.49 - 2.72 (m, 2H), 2.14 - 2.29 (m, 4H), 1.97 - 2.07 (m, 2H), 1.74 - 1.82 (m, 2H), 1.32 (d, *J =* 6.87 Hz, 3H). MS (ESI): [M + H]⁺ = 455.12

### (20) Synthesis of methyl 3-(4-((1-(chlorocarbonyl)-3-phenyl-1H-pyrazolo[4,3-b]pyridin-6-yl)methoxy)phenyl)butanoate (Compound J83)

Compound J83 was synthesized from J6 and acetyl chloride in the same manner as described for the synthesis of Compound J76, to afford a colorless oil (52% yield).

### (21) Synthesis of 3-(4-((1-(cyclohexylmethyl)-3-phenyl-1H-indazol-6-yl)methoxy)phenyl)butanoic acid (Compound 83)

Compound 83 was synthesized from Compound J82 in the same manner as described for the synthesis of Compound 76, to afford a white solid (85% yield).

¹H NMR (CDCl₃, 400 MHz): δ 8.01 (dd, *J* = 0.70, 8.50 Hz, 1H), 7.93 - 7.98 (m, 2H), 7.45 - 7.54 (m, 3H), 7.37 - 7.43 (m, 1H), 7.24 (dd, *J* = 1.37, 8.47 Hz, 1H), 7.15 - 7.20 (m, 2H), 6.94 - 7.00 (m, 2H), 5.22 (s, 2H), 4.25 (d, *J =* 7.33 Hz, 2H), 3.21 - 3.32 (m, 1H), 2.62 (dq, *J* = 7.56, 15.49 Hz, 2H), 2.02 - 2.14 (m, 1H), 1.47 - 1.71 (m, 10H), 1.29 - 1.33 (m, 3H), 0.80 - 0.93 (m, 1H). MS (ESI): [M + H]⁺ = 483.12

### (22) Synthesis of methyl 3-(4-((1-benzyl-3-phenyl-1H-pyrazolo[4,3-b]pyridin-6-yl)methoxy)phenyl)butanoate (Compound J84)

Compound J84 was synthesized from J6 and (bromomethyl)benzene in the same manner as described for the synthesis of Compound J76, to afford a colorless oil (41% yield).

### (23) Synthesis of 3-(4-((1-acetyl-3-phenyl-1H-pyrazolo[4,3-b]pyridin-6-yl)methoxy)phenyl)butanoic acid (Compound 84)

Compound 84 was synthesized from Compound J83 in the same manner as described for the synthesis of Compound 76, to afford a white solid (65% yield).

### (24) Synthesis of methyl 3-(4-((1-benzoyl-3-phenyl-1H-pyrazolo[4,3-b]pyridin-6-yl)methoxy)phenyl)butanoate (Compound J85)

Compound J85 was synthesized from J6 and benzoyl chloride in the same manner as described for the synthesis of Compound J76, to afford a colorless oil (31% yield).

### (25) Synthesis of (Compound 85)

Compound 85 was synthesized from Compound J84 in the same manner as described for the synthesis of Compound 76, to afford a white solid (71% yield).

### (26) Synthesis of (Compound 86)

Compound 86 was synthesized from Compound J85 in the same manner as described for the synthesis of Compound 76, to afford a white solid (59% yield).

### Experimental Example

### Experimental Example 1. Cell Line Establishment and Culture

To establish a CMKLR1 calcium mobilization cell evaluation system, CHO-K1 cells expressing Gα-16 were transfected with pcDNA3.1-derived human CMKLR1-expressing plasmids using Lipofectamine 2000 (Invitrogen, Waltham, Massachusetts, USA). Cells were selected using 0.8 mg/mL geneticin (Invitrogen), and CMKLR1 expression in the cells was confirmed by calcium mobilization using chemerin-9 (Genscript, New Jersey, USA). The established cell line (CHO-Gₐ16-CMKLR1) was cultured in F-12 medium (Invitrogen) supplemented with 10% (v/v) fetal bovine serum (FBS; Invitrogen), 0.5 mg/mL geneticin, 0.2 mg/mL hygromycin B (Clontech), and 1% (v/v) antibiotic-antimycotic solution (Invitrogen) under 5% CO₂ at 37°C.

### Experimental Example 2. Calcium Mobilization Assay

Cells were diluted with culture medium to a final concentration of 3 × 10⁵ cells/mL. 100 µL/well was added to a 96-well plate (Corning) and incubated for 24 h. The buffer solution was prepared by adding 1 M HEPES solution to Hanks' Balanced Salt Solution (HBSS) (Invitrogen) and adjusting the pH to 7.4 by dropwise addition of 1 M NaOH.

The fluorescent dye solution was prepared using the buffer provided with the Calcium 5 Assay Kit (Molecular Devices). 40 µL/well of dye solution was added to each well and incubated at 37°C in a 5% CO₂ atmosphere for 30 min. Thereafter, test compounds were diluted at various concentrations and diluted with buffer (0.00064 µM to 10 µM) to prepare 5× stock solutions. The final DMSO concentration was adjusted to 2%. 40 µL of each compound solution was added to the well and incubated for 30 min. Intracellular calcium concentration was measured using a FlexStation II384 (Molecular Devices) after adding 50 µL of 2.5 nM chemerin-9 (200 × dilution with buffer). The CMKLR1 inhibitory activity of the compounds was determined relative to the calcium fluorescence signal induced by 2.5 nM chemerin-9. Prism 5 software (GraphPad) was then used to obtain IC₅₀ values for various compounds. IC₅₀ values were derived based on concentration-response curves, and all experiments were repeated at least three times.

**[TABLE 1]**

| Compound | CMKLR1 activity in CHO cells (% inhibition at 10 µM) | Compound | CMKLR1 activity in CHO cells (% inhibition at 10 µM) |
|---|---|---|---|
| 3 | 10 | 80 | 52 |
| 7 | 81 | 81 | 35 |
| 8 | 25 | 82 | 94 |
| 9 | 81 | 83 | 28 |
| 77 | 24 | 84 | 27 |
| 78 | 66 | 85 | 23 |
| 79 | 60 | | |

**[TABLE 2]**

| Compound | CMKLR1 activity in CHO cells | Compound | CMKLR1 activity in CHO cells |
|---|---|---|---|
| 10 | * | 20 | ** |
| 11 | * | 21 | ** |
| 12 | ** | 22 | * |
| 13 | * | 23 | * |
| 14 | * | 24 | * |
| 15 | * | 25 | * |
| 16 | * | 26 | * |
| 17 | * | 27 | * |
| 18 | * | 28 | * |
| 19 | * | 29 | * |
| IC₅₀<100 nM, ***; 100 nM≤IC₅₀<1000 nM, **; 1000 nM<IC₅₀, * | | | |

**[TABLE 3]**

| Compound | CMKLR1 activity in CHO cells | Compound | CMKLR1 activity in CHO cells |
|---|---|---|---|
| 30 | ** | 40 | * |
| 31 | * | 41 | ** |
| 32 | * | 42 | ** |
| 33 | * | 43 | ** |
| 34 | ** | 44 | ** |
| 35 | ** | 45 | ** |
| 36 | ** | 46 | ** |
| 37 | * | 47 | *** |
| 38 | * | 48 | *** |
| 39 | * | 51 | ** |
| IC₅₀<100 nM, ***; 100 nM≤IC₅₀<1000 nM, **; 1000 nM<IC₅₀, * | | | |

**[TABLE 4]**

| Compound | CMKLR1 activity in CHO cells | Compound | CMKLR1 activity in CHO cells |
|---|---|---|---|
| 52 | ** | 68 | ** |
| 53 | ** | 69 | *** |
| 54 | * | 70 | ** |
| 55 | * | 71 | *** |
| 56 | * | 72 | ** |
| 57 | * | 73 | ** |
| 58 | * | 74 | ** |
| 59 | * | 75 | ** |
| 60 | * | | |
| 61 | * | | |
| IC₅₀<100 nM, ***; 100 nM≤IC₅₀<1000 nM, **; 1000 nM<IC₅₀, * | | | |

### Experimental Example 3. Confirmation of Psoriasis Treatment Effect (In Vivo)

To establish a psoriasis-induced mouse model, 8 to 10-week-old female BALB/c mice were used. Six mice were assigned to each group, as follows: the normal group (no treatment, Normal), the control group (psoriasis induced with imiquimod, Control), the MTX group (psoriasis induced with imiquimod followed by methotrexate at 0.4 mg/kg, MTX), and the test drug groups (psoriasis induced with imiquimod followed by Compound 71, 30d (2 mpk) and 30d (30 mpk)). After shaving the backs of the mice, 65 mg of imiquimod was evenly applied to the backs for seven consecutive days. Methotrexate and Compound 71 were administered orally. The severity of psoriasis lesions was assessed using PASI scoring, which assessed erythema, scaliness, and thickness of the psoriasis lesions in each mouse on a scale from 0 (normal) to 4 (severe). After the experiment ended on day 7, tissue samples from each mouse were collected for staining evaluation.

## Claims

1. A compound represented by Formula 1 below or a pharmaceutically acceptable salt thereof: 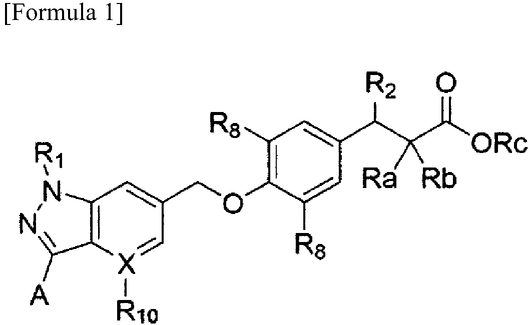
(in Formula 1, A is a substituted or unsubstituted 5- to 10-membered aryl or heteroaryl;
R₁ is C₁ to C₄ alkyl, C₃ to C₆ cycloalkyl, acetyl, benzyl, or methyl substituted with C₃ to C₆ cycloalkyl;
R₂ is C₁ to C₄ alkyl or C₃ to C₆ cycloalkyl;
R₈ is hydrogen or halogen;
Rₐ and R_{b} are each independently hydrogen or methyl;
R_{c} is hydrogen or C₁ to C₄ alkyl;
X is carbon or nitrogen; and R₁₀ is absent, hydrogen, halogen, or methyl unsubstituted or substituted with halogen.

2. The compound or a pharmaceutically acceptable salt thereof according to claim 1, wherein A is substituted or unsubstituted phenyl, pyridine, benzo[b]thiophene, or 1H-tetrazole.

3. The compound or a pharmaceutically acceptable salt thereof according to claim 1, wherein the substituent of A is C₁ to C₃ alkyl, C₁ to C₅ alkoxy, -OH, -CF₃, -CN, -CH₂OH, -NH₂, -CH=NOH, halogen, phenyl, -NO₂, -OCH₂CH=CHC(=O)OH, -OCH₂CH=CH₂, - C(=O)OH, -NHC(=O)R₉, or dioxane, and
R₉ is C₂ to C₄ alkyl or alkenyl.

4. The compound or a pharmaceutically acceptable salt thereof according to claim 1, wherein A is substituted or unsubstituted phenyl, and the substituent is selected from the group consisting of C₁ to C₃ alkyl, C₁ to C₅ alkoxy, -OH, -CF₃, -CN, -CH₂OH, -NH₂, - CH=NOH, halogen, phenyl, -NO₂, -OCH₂CH=CHC(=O)OH, -OCH₂CH=CH₂, -C(=O)OH, -NHC(=O)R₉ (wherein R₉ is C2 to C4 alkyl or alkenyl), or dioxane.

5. The compound or a pharmaceutically acceptable salt thereof according to claim 1, wherein the compound represented by Formula 1 is any one selected from the group consisting of the following compounds:
| Compound | Structure | Compound | Structure |
|---|---|---|---|
| 1 | 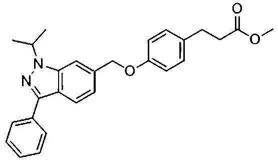 | 2 | 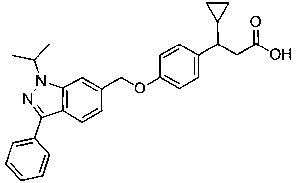 |
| 3 | 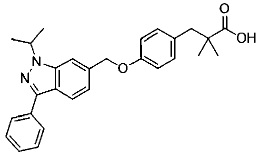 | 4 | 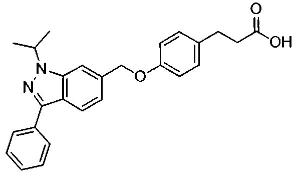 |
| 5 | 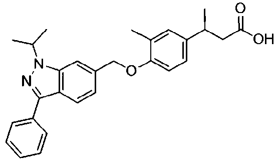 | 6 | 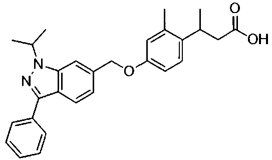 |
| 7 | 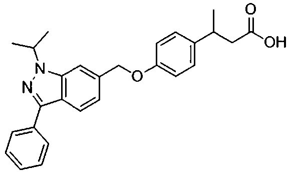 | 8 | 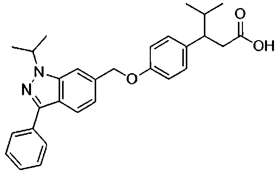 |
| 9 | 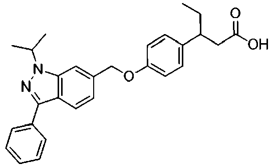 | 10 | 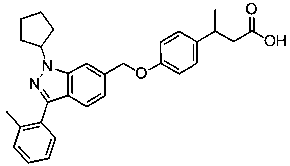 |
| Compound | Structure | Compound | Structure |
|---|---|---|---|
| 11 | 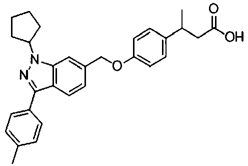 | 12 | 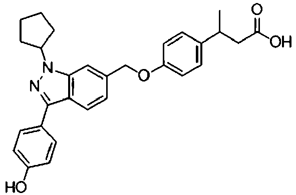 |
| 13 | 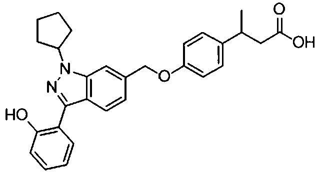 | 14 | 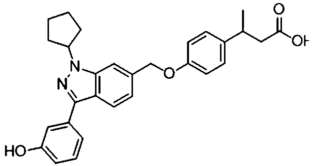 |
| 15 | 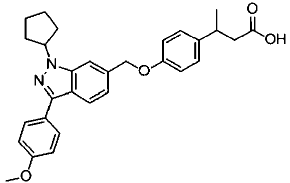 | 16 | 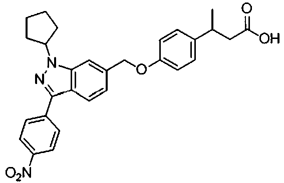 |
| 17 | 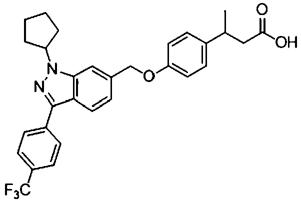 | 18 | 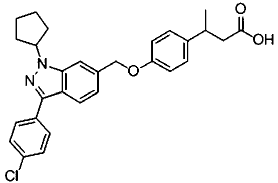 |
| 19 | 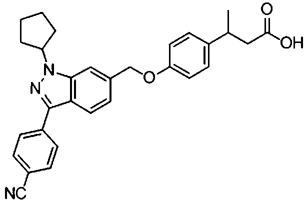 | 20 | 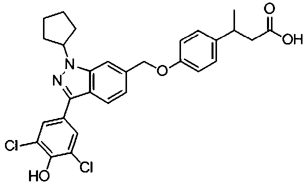 |
| Compound | Structure | Compound | Structure |
|---|---|---|---|
| 21 | 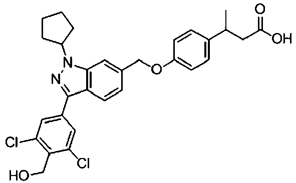 | 22 | 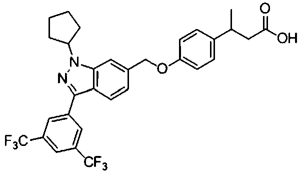 |
| 23 | 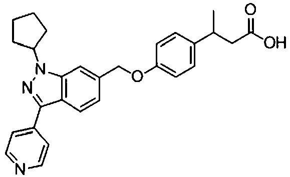 | 24 | 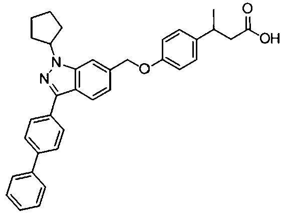 |
| 25 | 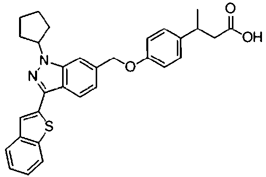 | 26 | 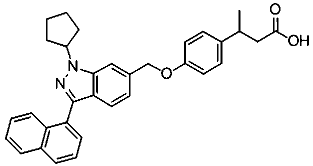 |
| 27 | 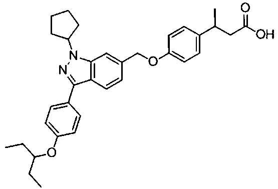 | 28 | 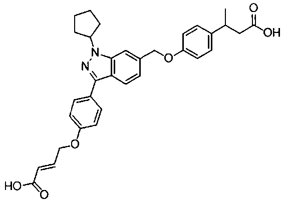 |
| 29 | 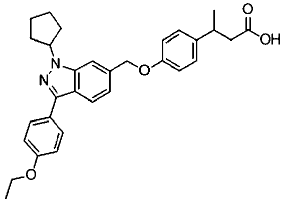 | 30 | 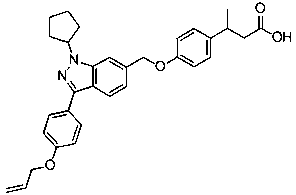 |
| Compound | Structure | Compound | Structure |
|---|---|---|---|
| 31 | 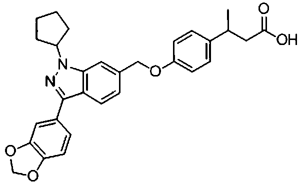 | 32 | 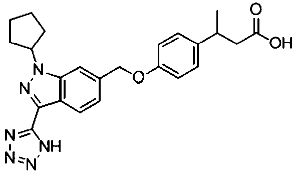 |
| 33 | 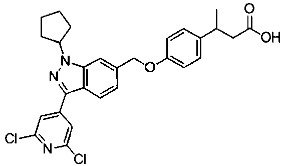 | 34 | 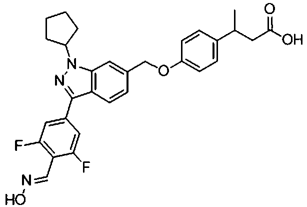 |
| 35 | 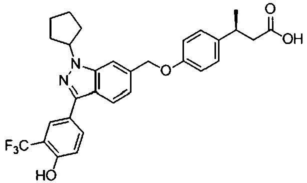 | 36 | 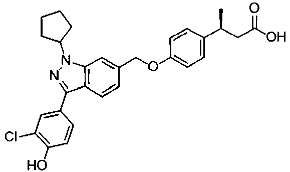 |
| 37 | 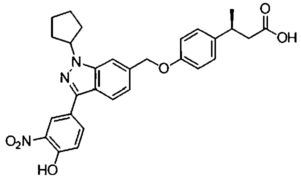 | 38 | 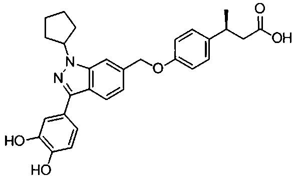 |
| 39 | 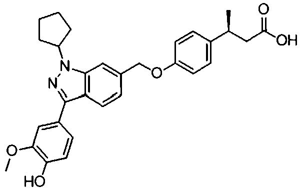 | 40 | 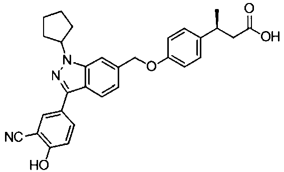 |
| Compound | Structure | Compound | Structure |
|---|---|---|---|
| 41 | 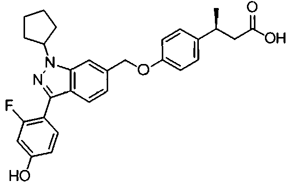 | 42 | 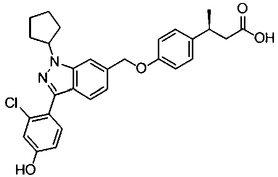 |
| 43 | 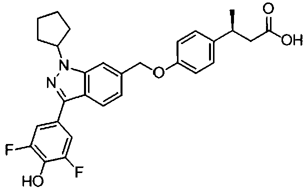 | 44 | 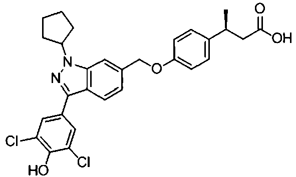 |
| 45 | 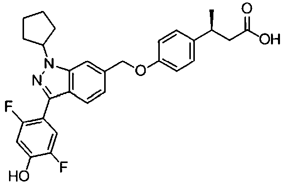 | 46 | 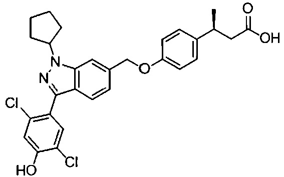 |
| 47 | 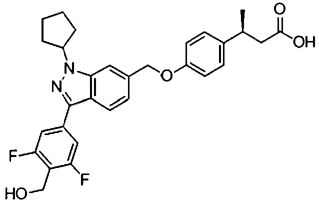 | 48 | 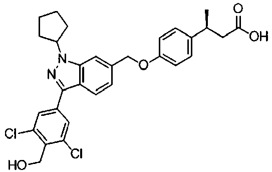 |
| 49 | 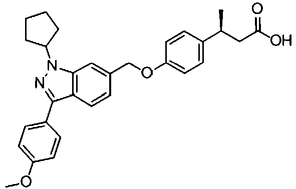 | 50 | 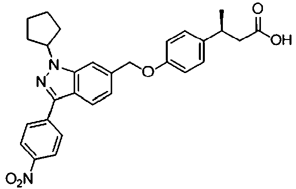 |
| Compound | Structure | Compound | Structure |
|---|---|---|---|
| 51 | 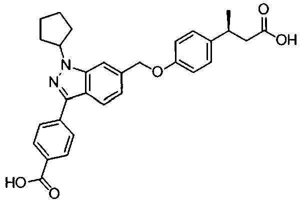 | 52 | 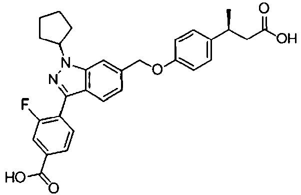 |
| 53 | 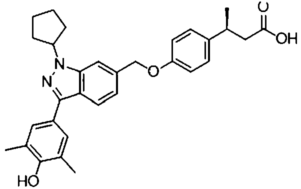 | 54 | 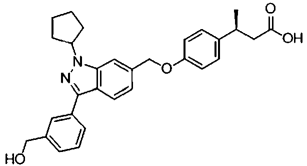 |
| 55 | 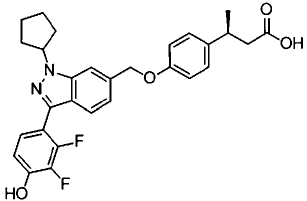 | 56 | 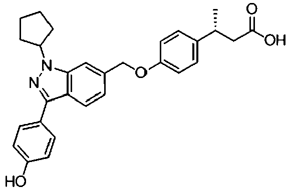 |
| 57 | 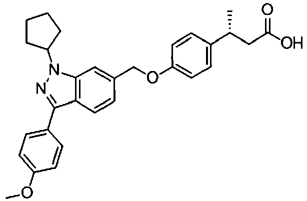 | 58 | 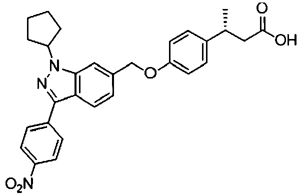 |
| 59 | 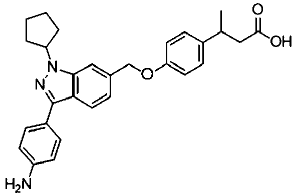 | 60 | 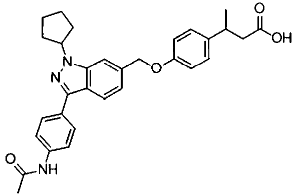 |
| Compound | Structure | Compound | Structure |
|---|---|---|---|
| 61 | 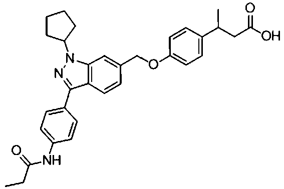 | 62 | 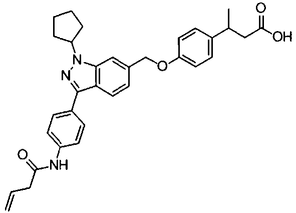 |
| 63 | 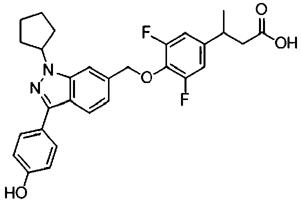 | 64 | 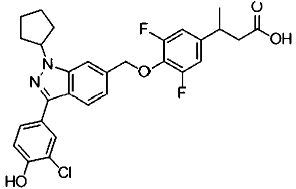 |
| 65 | 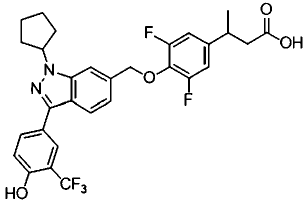 | 66 | 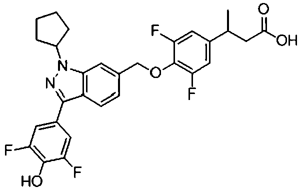 |
| 67 | 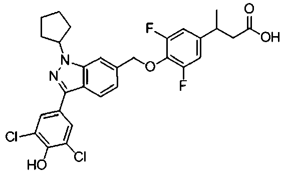 | 68 | 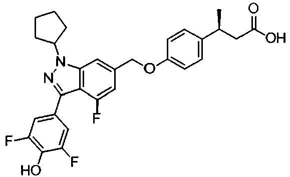 |
| 69 | 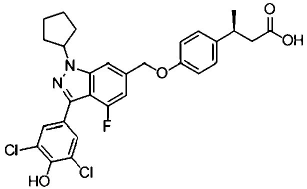 | 70 | 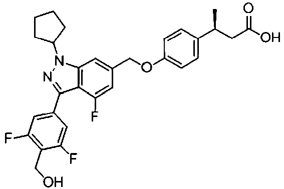 |
| Compound | Structure | Compound | Structure |
|---|---|---|---|
| 71 | 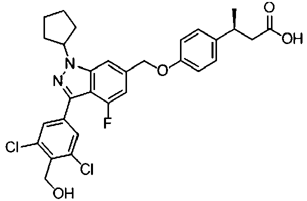 | 72 | 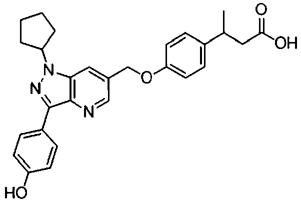 |
| 73 | 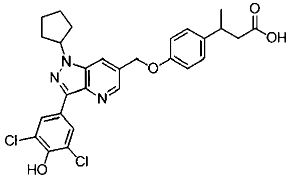 | 74 | 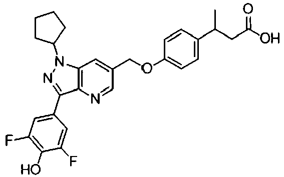 |
| 75 | 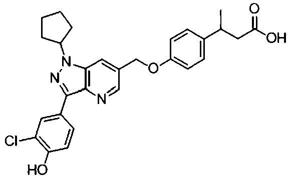 | 76 | 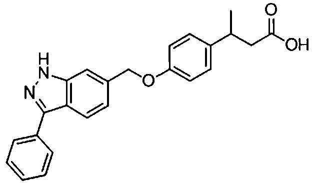 |
| 77 | 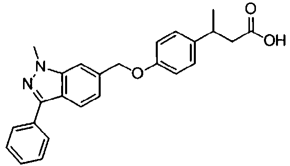 | 78 | 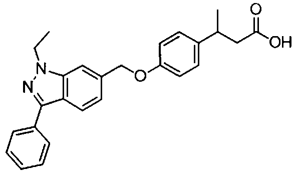 |
| 79 | 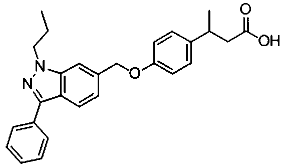 | 80 | 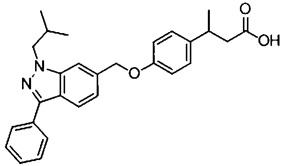 |
| Compound | Structure | Compound | Structure |
|---|---|---|---|
| 81 | 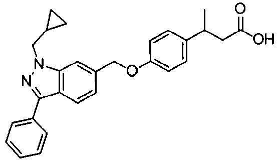 | 82 | 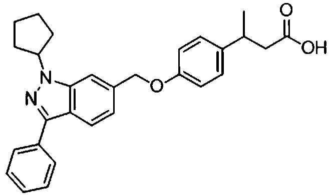 |
| 83 | 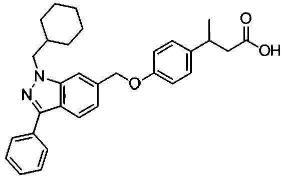 | 84 | 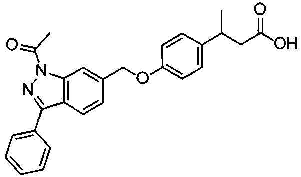 |
| 85 | 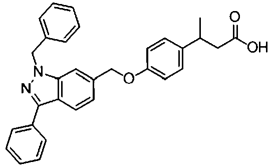 | 86 | 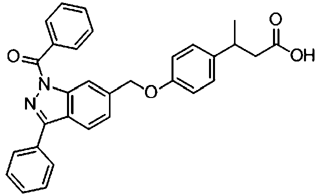 |

6. A pharmaceutical composition for preventing or treating psoriasis, comprising the compound of any one of claims 1 to 5 or a pharmaceutically acceptable salt thereof.
